# EUROPÄISCHE PATENTSCHRIFT

(11) **EP 1 565 450 B1**
(45) Veröffentlichungstag und Bekanntmachung des Hinweises auf die Patenterteilung: **04.07.2007**
(21) Anmeldenummer: 03779946.7
(22) Anmeldetag: 17.11.2003
(51) Int. Cl.: C07D 309/30, C07D 409/04, C07D 309/20, C07D 309/06, C07D 409/08, C07D 309/08, C07D 309/04

(54) **TETRAHYDROPYRAN-DERIVATE**
TETRAHYDROPYRAN DERIVATIVES
DERIVES DE TETRAHYDROPYRANE

(30) Priorität: 27.11.2002 DE 10255312
(43) Veröffentlichungstag der Anmeldung: 24.08.2005
(73) Patentinhaber: Merck Patent GmbH, 64293 Darmstadt (DE)
(72) Erfinder: KIRSCH, Peer, 64342 Seeheim-Jugenheim (DE); HAHN, Alexander, 65428 Rüsselsheim (DE); POETSCH, Eike, 64367 Mühtal (DE); BINDER, Werner, 64807 Dieburg (DE); MEYER, Volker, 64846 Gross-Zimmern (DE)
(86) Internationale Anmeldenummer: PCT/EP2003/012812
(87) Internationale Veröffentlichungsnummer: WO 2004/048357

(56) Entgegenhaltungen:
- EP-A- 0 594 861
- EP-A- 0 684 246
- EP-A- 0 700 983
- DE-A- 4 132 006
- US-A- 4 818 431
- US-A- 5 958 290
- KIRSCH P ET AL: "DIFLUOROOXYMETHYLENE-BRIDGED LIQUID CRYSTALS: A NOVEL SYNTHESIS BASED ON THE OXIDATIVE ALKOXYDIFLUORODESULFURATION OF DITHIANYLIUM SALTS" ANGEWANDTE CHEMIE. INTERNATIONAL EDITION, VERLAG CHEMIE. WEINHEIM, DE, Bd. 40, Nr. 8, 2001, Seiten 1480-1484, XP001030024 ISSN: 0570-0833

## Beschreibung

Die Erfindung betrifft Tetrahydropyran-Derivate und Verfahren zu ihrer Herstellung.

Tetrahydropyran-Derivate spielen eine bedeutende Rolle in Chemie und Pharmazie, unter anderem als Inhaltsstoffe von natürlichen und synthetischen Aromastoffen, in Arzneimitteln und in flüssigkristallinen Materialien. Allerdings ist der präparative Zugang zu Tetrahydropyranen gegenwärtig limitiert und beschränkt sich zumeist auf die Derivatisierung von Kohlenhydraten, die Pyranoseringeinheiten aufweisen.

Der vorliegenden Erfindung liegt daher als eine Aufgabe zugrunde, Tetrahydropyran-Derivate bereitzustellen, die sich als Synthone für den Aufbau komplexer, Tetrahydropyraneinheiten enthaltender Moleküle eignen.

Diese Aufgabe wird gelöst durch die Bereitstellung von Verbindungen der allgemeinen Formel I 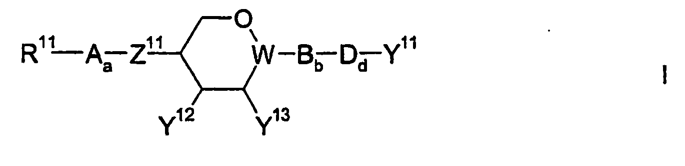 worin
- R¹¹: H, F, Cl, Br, I, CN, Aryl, Heterocyclyl oder einen halogenierten oder unsubstituierten Alkylrest mit 1 bis 15 Kohlenstoffatomen bedeutet, wobei in diesem Rest auch eine oder mehrere CH₂-Gruppen jeweils unabhängig voneinander durch -C≡C-, -CH=CH-, -O-, -CO-, -CO-O- oder -O-CO- so ersetzt sein können, daß O-Atome nicht direkt miteinander verknüpft sind;
- A: für 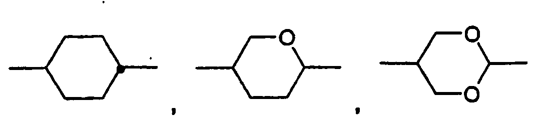 oder 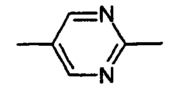 steht;
- a: 0, 1 oder 2 ist, wobei A gleiche oder verschiedene Bedeutungen annehmen kann, wenn a 2 ist;
- Z¹¹: eine Einfachbindung, -CH₂-CH₂-, -CF₂-CF₂-, -CFrCH₂-, -CH₂-CF₂-, -CH₂-O-, -O-CH₂-, -CF₂-O- oder -O-CF₂- darstellt;
- W: >CH- oder >C= bedeutet;
- B und D: unabhängig voneinander für 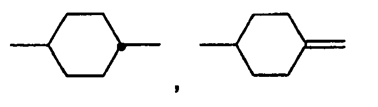 oder 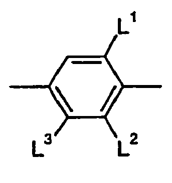 stehen;
- b und d: unabhängig voneinander 0 oder 1 sind;
- Y¹¹: =O, =C(SR¹²)(SR¹³), =CF₂, -H, -F, -Cl, -Br, -I, -CN, -OH, -SH, -CO-R¹⁴, -OSO₂R¹⁵, -C(=S⁺R¹²)(-SR¹³)X⁻, -B(OR¹⁶)(OR¹⁷), -BF₃⁻Kat⁺, -Si(OR¹⁸)(OR¹⁹)(OR²⁰) oder Alkyl bedeutet, wobei Alkyl einen halogenierten oder unsubstituierten Alkylrest mit 1 bis 15 C-Atomen bedeutet, in welchem auch eine oder mehrere CH₂-Gruppen jeweils unabhängig voneinander durch -C≡C-, -CH=CH-, -O-, -CO-, -CO-O- oder -O-CO- so ersetzt sein können, daß O-Atome nicht direkt miteinander verknüpft sind;
- Y¹² und Y¹³: unabhängig voneinander H oder Alkyl bedeuten, wobei Alkyl einen halogenierten oder unsubstituierten Alkylrest mit 1 bis 15 C-Atomen bedeutet, in welchem auch eine oder mehrere CH₂-Gruppen jeweils unabhängig voneinander durch -C≡C-, -CH=CH-, -O-, -CO-, -CO-O- oder -O-CO- so ersetzt sein können, daß O-Atome nicht direkt miteinander verknüpft sind;
- L¹, L² und L³: unabhängig voneinander H oder F bedeuten;
- R¹² und R¹³: unabhängig voneinander einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können;
- R¹⁴: OH, O-Aryl, O-Aralkyl, O-Alkyl, Cl, Br, Aryl, Aralkyl oder Alkyl bedeutet;
- R¹⁵: Aryl, Aralkyl oder einen halogenierten oder unsubstituierten Alkylrest mit 1 bis 15 Kohlenstoffatomen bedeutet, wobei in diesem Alkylrest auch eine oder mehrere CH₂-Gruppen jeweils unabhängig voneinander durch -C≡C-, -CH=CH-, -O-, -CO-, -CO-O- oder -O-CO- so ersetzt sein können, daß O-Atome nicht direkt miteinander verknüpft sind;
- R¹⁶: und R¹⁷ H oder einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können;
- R¹⁸, R¹⁹ und R²⁰: unabhängig voneinander einen unverzweigten oder verzweigten Alkylrest mit 1 bis 15 Kohlenstoffatomen bedeuten;
- Kat⁺: ein Alkalimetallkation oder ein quartäres Ammoniumkation ist; und
- X⁻: ein schwach koordinierendes Anion ist;
mit der Maßgabe,
daß W >CH- bedeutet, wenn b+d ≠ 0;
daß Y¹¹ nicht =O, =C(SR¹²)(SR¹³) oder =CF₂ bedeutet, wenn Y¹¹ mit B oder 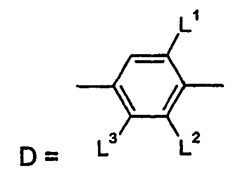 verbunden ist;
daß Y¹¹ -H, -I, -OH, -SH, -CO₂R¹⁴, -OSO₂R¹⁵, -C(=S⁺R¹²)(SR¹³)X⁻, -B(OR¹⁶)(OR¹⁷), -BF₃⁻Kat⁺, -Si(OR¹⁸)(OR¹⁹)(OR²⁰) oder Alkyl bedeutet (wobei Alkyl einen halogenierten oder unsubstituierten Alkylrest mit 1 bis 15 C-Atomen bedeutet, in welchem eine oder mehrere CH₂-Gruppen jeweils unabhängig voneinander durch -C≡C-, -CH=CH-, -O-, -CO-, -CO-O- oder -O-CO- so ersetzt sind, daß O-Atome nicht direkt miteinander verknüpft sind und Alkyl nicht für Alkoxy steht), wenn W direkt verbunden ist mit 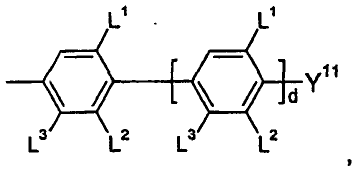 wobei d 0 oder 1 ist;
und
daß B nicht für 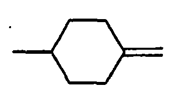 steht, wenn d = 1.

Die erfindungsgemäßen Verbindungen sind nützlich als Zwischenverbindungen zur Herstellung von komplexeren Molekülen, die eine Tetrahydropyraneinheit als Molekülbestandteil aufweisen und beispielsweise eine Verwendung als Aromastoff, Arzneimittel und Komponente von insbesondere flüssigkristallinen Mischungen für den Einsatz in elektrooptischen Vorrichtungen finden.

Die Begriff "Alkyl" umfaßt - sofern er nicht an anderer Stelle dieser Beschreibung oder in den Ansprüchen abweichend definiert ist - gesättigte und ungesättigte nicht-cyclische (aliphatische) Kohlenwasserstoffreste, die jeweils unsubstituiert oder mit Halogen substituiert sein können.

"Alkyl" umfaßt dabei u.a. geradkettige und verzweigte gesättigte Alkylgruppen (Alkanyle) mit 1-15, vorzugsweise 1, 2, 3, 4, 5, 6 oder 7 Kohlenstoffatomen, insbesondere die geradkettigen Gruppen Methyl, Ethyl, Propyl, Butyl, Pentyl, Hexyl und Heptyl, ferner Octyl, Nonyl, Decyl, Undecyl, Dodecyl, Tridecyl, Tetradecyl und Pentadecyl. Gruppen mit 2-5 Kohlenstoffatomen sind im allgemeinen bevorzugt. Ferner umfaßt der Ausdruck "Alkyl" mit Fluor, Chlor, Brom und/oder Iod einfach oder mehrfach halogenierte Kohlenwasserstoffreste, wie z.B. -CF₃, -CHF₂, -CH₂F. Bevorzugte verzweigte Alkylreste sind Isopropyl, 2-Butyl, Isobutyl, tert.-Butyl, 2-Methylbutyl, Isopentyl, 2-Methylpentyl, 3-Methylpentyl, 2-Ethylhexyl und 2-Propylpentyl.

In seiner allgemeinsten Bedeutung umfaßt der Begriff "Alkyl" u.a. auch Alkenylreste, d.h. wenigstens eine CH₂-Gruppe des Alkylrests ist durch -CH=CH- ersetzt, wobei die -CH=CH-Gruppe auch endständig als H-CH=CH- vorliegen beziehungsweise jede der CH-Einheiten auch mit Halogen substituiert sein kann (wie z.B. in -CF=CH- oder -CF=CF-); und Alkinylreste, d.h. wenigstens eine CH₂-Gruppe des Alkylrestes ist durch -C≡C- ersetzt, wobei die -C≡C-Gruppe auch endständig als H-C≡Cvorliegen kann. Alkenyl- und Alkinylreste weisen unabhängig voneinander 2 bis 15 Kohlenstoffatome, vorzugsweise 2, 3, 4, 5, 6 oder 7 Kohlenstoffatome auf. Die Alkenylreste können als E- und/oder Z-Isomeren vorliegen, wobei im allgemeinen das jeweilige E-Isomere bevorzugt ist. Bevorzugte Alkenylgruppen sind H₂C=CH-, CH₃-CH=CH- und C₂H₅-CH=CH-, eine bevorzugte Alkinylgruppe ist H₃C-C≡C-.

Ferner umfaßt der Ausdruck "Alkyl" im Sinne dieser Erfindung auch solche Reste, in denen eine oder mehrere CH₂-Gruppen durch O so ersetzt sind, daß die Sauerstoffatome nicht unmittelbar miteinander verbunden sind, d.h. Alkoxy- und Oxaalkylreste, die 1 bis 15, bevorzugt 1, 2, 3, 4, 5, 6 oder 7 Kohlenstoffatome aufweisen und besonders bevorzugt unverzweigt sind. Bevorzugte Alkoxyreste sind Methoxy, Ethoxy, Propoxy, Butoxy, Pentoxy, Hexoxy, Heptoxy sowie (per-)fluorierte Alkoxyreste wie -OCH₂F, -OCHCF₂, -OCF₃, -OCH₂CF₃ oder -OCF₂CF₃, ferner Octoxy, Nonoxy, Decoxy, Undecoxy, Dodecoxy, Tridecoxy, Tetradecoxy. Bevorzugte verzweigte Alkoxyreste sind Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy. Bevorzugte Oxaalkylreste sind 2-Oxapropyl (= Methoxymethyl), 2-Oxabutyl (= Ethoxymethyl), 3-Oxabutyl, 2-, 3- oder 4-Oxapentyl, 2-, 3-, 4- oder 5-Oxahexyl, 2-, 3-, 4-, 5- oder 6-Oxaheptyl; ferner u.a. 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Der Ausdruck "Alkyl" umfaß auch Reste, in denen eine CH₂-Gruppe durch -CO- ersetzt ist. Vorzugsweise sind diese Reste geradkettig und weisen 2 bis 7 Kohlenstoffatome auf.

Darüber hinaus umfaßt der Ausdruck "Alkyl" im Sinne der vorliegenden Erfindung auch Reste, in denen eine CH₂-Gruppe durch -O- und eine durch -CO- ersetzt ist, wobei diese bevorzugt benachbart sind. Somit schließen diese eine Acyloxygruppe -CO-O- und eine Oxycarbonylgruppe -O-CO- ein. Vorzugsweise sind diese geradkettig und haben 2 bis 7 Kohlenstoffatome. Sie bedeuten demnach insbesondere Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, Hexanoyloxymethyl, 2-Acetyloxyethyl, 2-Propionyloxyethyl, 2-Butyryloxyethyl, 2-Acetoxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl oder 4-(Methoxycarbonyl)butyl.

Ferner umfaßt "Alkyl" im Sinne der vorliegenden Erfindung auch einen solchen Rest, in dem eine CH₂-Gruppe durch unsubstituiertes oder substituiertes -CH=CH- und eine benachbarte CH₂-Gruppe durch CO oder CO-O oder O-CO ersetzt ist, wobei dieser Rest geradkettig oder verzweigt sein kann. Vorzugsweise ist er geradkettig und weist 4 bis 12 Kohlenstoffatome auf. Er bedeutet demnach insbesondere Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Der Ausdruck "Alkyl" umfaßt im Sinne dieser Erfindung auch Reste, in denen zwei oder mehr CH₂-Gruppen durch -O- und/oder -CO-O- ersetzt sind, wobei diese geradkettig oder verzweigt sein können. Vorzugsweise sind diese Reste verzweigt und haben 3 bis 12 Kohlenstoffatome. Sie bedeuten demnach insbesondere Biscarboxymethyl, 2,2-Biscarboxyethyl, 3,3-Biscarboxypropyl, 4,4-Biscarboxybutyl, 5,5-Biscarboxypentyl, 6,6-Biscarboxyhexyl, 7,7-Biscarboxyheptyl, 8,8-Biscarboxyoctyl, 9,9-Biscarboxynonyl, 10,10-Biscarboxydecyl, Bis-(methoxycarbonyl)methyl, 2,2-Bis-(methoxycarbonyl)ethyl, 3,3-Bis-(methoxycarbonyl)propyl, 4,4-Bis-(methoxycarbonyl)butyl, 5,5-Bis-(methoxycarbonyl)pentyl, 6,6-Bis-(methoxycarbonyl)hexyl, 7,7-Bis-(methoxycarbonyl)heptyl, 8,8-Bis-(methoxycarbonyl)octyl, Bis-(ethoxycarbonyl)methyl, 2,2-Bis-(ethoxycarbonyl)ethyl, 3,3-Bis-(ethoxycarbonyl)propyl, 4,4-Bis-(ethoxycarbonyl)-butyl, 5,5-Bis-(ethoxycarbonyl)hexyl.

Der Ausdruck "Aryl" umfaßt - soweit er nicht an anderer Stelle der Beschreibung oder in den Ansprüchen abweichend definiert ist - im Sinne der vorliegenden Erfindung aromatische Kohlenwasserstoffreste mit vorzugsweise 6 bis 18 Kohlenstoffatomen, die gegebenenfalls mit F, Cl, Br, I, CN, OH, SH, O-Alkyl, S-Alkyl, COOH, COOAlkyl, Amino, NO₂ oder Alkyl einfach oder mehrfach gleich oder verschieden substituiert sind. Vorzugsweise steht "Aryl" für einen Phenyl- oder Naphthylrest, jeweils optional in 4-Position substituiert mit Alkyl, oder für einen Biphenylrest.

Der Ausdruck "Heterocyclyl" umfaßt - soweit er nicht an anderer Stelle der Beschreibung oder in den Ansprüchen abweichend definiert ist - im Sinne der vorliegenden Erfindung heterocyclische gesättigte und ungesättigte, unter anderem auch heteroaromatische Reste, in denen wenigstens ein Ringatom ein Heteroatom ist, ausgewählt aus der Gruppe, die aus O, S und N besteht. Der Heterocyclyl-Rest ist gegebenenfalls mit F, Cl, Br, I, CN, OH, SH, O-Alkyl, S-Alkyl, COOH, COOAlkyl, Amino, NO₂, Aryl oder Alkyl einfach oder mehrfach gleich oder verschieden substituiert. Bevorzugt steht Heterocyclyl für Dioxanyl, Furanyl, Thienyl, Pyranyl, Pyrrolidinyl, Piperidinyl, Morpholinyl und Pyrimidinyl. Die Verknüpfung des Heterocyclylrests mit einem Atom, einer Kette, einem Ring oder einem anderen Molekülbestandteil kann im allgemeinen über jedes Ringatom erfolgen.

Der Ausdruck "Aralkyl" umfaßt - soweit er nicht an anderer Stelle der Beschreibung oder in den Ansprüchen abweichend definiert ist - im Sinne der vorliegenden Erfindung Kohlenwasserstoffreste mit einem Arylbestandteil und einer Alkylbrücke, wie z.B. Benzyl oder Phenethyl (Phenyl-CH₂-CH₂-), Dabei kann insbesondere der Arylbestandteil so, wie oben für "Aryl" definiert, substituiert sein. Besonders bevorzugt steht "Aralkyl" für Benzyl und Phenethyl. Ein "O-Aralkyl"-Rest ist für die Zwecke dieser Erfindung ein Aryl-Alkyl-O-Rest, d.h. die Verknüpfung mit einem weiteren Atom, einer weiteren Kette, einem weiteren Ring oder einem anderen Molekülbestandteil erfolgt über eine Alkyl-Sauerstoff-Brücke. Ein Beispiel für O-Aralkyl ist -O-Benzyl (-O-CH₂-Phenyl).

Halogen bedeutet im Zusammenhang der vorliegenden Erfindung Fluor, Chlor, Brom und/oder Iod.

In den erfindungsgemäßen Verbindungen der Formel I bedeutet A 1,4-Cyclohexylen, 2,5-Tetrahydropyranylen, 2,5-Dioxanylen oder 2,5-Pyrimidinylen, wobei die gesättigten Cyclen vorzugsweise trans verknüpft sind. In Abhängigkeit von der Bedeutung von a - das 0, 1 oder 2 ist - ist A in den erfindungsgemäßen Verbindungen der Formel I nicht vorhanden (wenn a = 0) oder einfach vorhanden (wenn a = 1) oder zweifach vorhanden (wenn a = 2). Falls a = 2, so kann A zweimal die gleiche

Bedeutung haben, z.B. 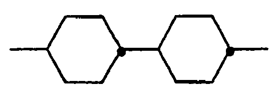 bedeuten, oder für verschiedene Ringe stehen, z.B. 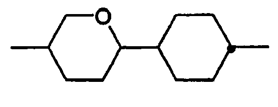 bedeuten. In einer bevorzugten Ausführungsform der vorliegenden Erfindung bedeutet A in Formel I 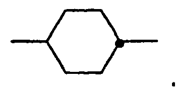 A steht vorzugsweise dann für 1,4-Cyclohexylen, wenn Z¹¹ eine Einfachbindung darstellt oder -CF₂O- oder -OCF₂- bedeutet. In einer weiteren bevorzugten Ausführungsform der Erfindung weisen die erfindungsgemäßen Verbindungen keinen Ring A auf, d.h. a = 0.

In den erfindungsgemäßen Verbindungen der Formel I stellt Z¹¹ eine Einfachbindung, -CH₂-CH₂-, -CF₂-CF₂-, -CF₂-CH₂-, -CHrCF₂-, -CH₂-O-, -O-CH₂-, -CF₂-O- oder -O-CF₂- dar. Es ist bevorzugt, daß Z¹¹ eine Einfachbindung darstellt oder -CF₂O- oder -OCF₂- bedeutet. Insbesondere steht Z¹¹ für eine Einfachbindung, so daß A beziehungsweise R¹¹ (wenn a = 0) unmittelbar mit dem zentralen Tetrahydropyranring verbunden ist.

In den erfindungsgemäßen Verbindungen der Formel I bedeutet R¹¹ H, F, Cl, Br, I, CN, Aryl, Heterocyclyl oder einen wie oben definierten Alkylrest. Bevorzugt ist R¹¹ ein unverzweigter halogenierter oder unsubstituierter Alkanylrest mit 1 bis 7 Kohlenstoffatomen, insbesondere CF₃-, CF₃-CF₂-, CF₃-CH₂-, Methyl, Ethyl, Propyl, Butyl, Pentyl, Hexyl oder Heptyl.

Die Substituenten des zentralen Tetrahydropyranrings, Y¹² und Y¹³, bedeuten unabhängig voneinander H oder ein wie oben definiertes Alkyl. Es ist bevorzugt, daß beide Substituenten Y¹² und Y¹³ Wasserstoff bedeuten, so daß der zentrale Tetrahydropyranring der erfindungsgemäßen Verbindungen 2,5-difunktionalisiert ist.

In den erfindungsgemäßen Verbindungen der Formel I steht W für >CH- oder für >C=. Steht W für >CH-, so ist W mit dem Rest B oder D beziehungsweise mit Y¹¹ über eine Einfachbindung verknüpft. Falls W für >C= steht, so gilt b = d = 0, und W ist mit Y¹¹ über eine Doppelbindung verknüpft.

Die Ringe B und D bedeuten in den erfindungsgemäßen Verbindungen der Formel I unabhängig voneinander 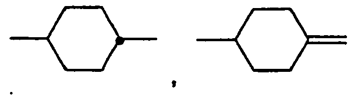 oder 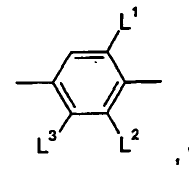 wobei B nicht 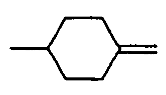 bedeutet, wenn auch ein Ring D vorhanden ist, d.h. wenn b = d = 1. In Abhängigkeit von der Bedeutung der Indizes b und d, die jeweils unabhängig voneinander 0 oder 1 bedeuten können, liegen die Verbindungen der Formel I vorzugsweise als 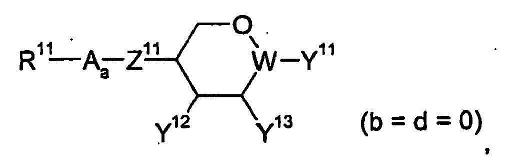 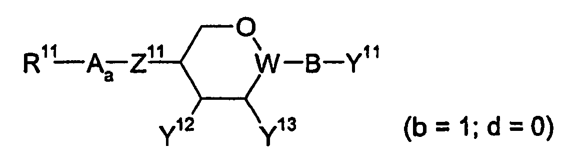 oder als 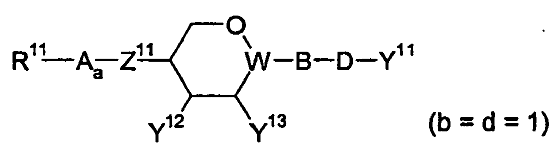 vor.

Wenn b = d = 0, so steht W bevorzugt für >C=. Falls einer der Ringe B und D für einen gegebenenfalls mit L¹, L² und/oder L³ substituierten 1,4-Phenylenring steht, so ist es bevorzugt, daß L¹, L² und L³ alle für Wasserstoff stehen oder L¹ und L² Fluor und L³ Wasserstoff bedeuten.

In den erfindungsgemäßen Verbindungen der Formel 1 bedeutet Y¹¹ dann =O, =C(SR¹²)(SR¹³) oder =CF₂, wenn Y¹¹ mit W = >C= verknüpft oder mit B beziehungsweise 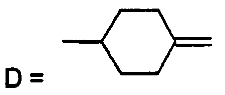 verbunden ist. Sofern Y¹¹ für =C(SR¹²)(SR¹³) steht, bedeuten R¹² und R¹³ unabhängig voneinander einen unverzweigten oder verzweigten Alkylrest mit 1 bis 15 Kohlenstoffatomen oder bilden zusammen eine Einheit -(CH₂)ₚ- mit p = 2, 3, 4, 5 oder 6, wobei eine, zwei oder drei dieser CH₂-Gruppen mit wenigstens einem unverzweigten oder verzweigten Alkylrest mit 1 bis 8 Kohlenstoffatomen substituiert sein können. Es ist bevorzugt, daß R¹² und R¹³ entweder die gleiche Bedeutung haben, also z.B. beide für Ethyl oder n-Propyl stehen, oder zusammen eine wie oben definierte Einheit -(CH₂)ₚ- bilden, die gegebenenfalls mit Alkylresten substituiert ist. Besonders bevorzugt stehen R¹² und R¹³ zusammen für -CHrCH₂-CHr oder -CH₂-C(CH₃)₂-CH₂-.

In den erfindungsgemäßen Verbindungen der Formel I steht Y¹¹ dann für -H, -F, -Cl, -Br, -I, -CN, -OH, -SH, -CO-R¹⁴, -OSO₂R¹⁵, -C(=S⁺R¹²)(-SR¹³)X⁻, -B(OR¹⁶)(OR¹⁷), -BF₃⁻Kat⁺, -Si(OR¹⁸)(OR¹⁹)(OR²⁰) oder ein wie oben definiertes Alkyl, wenn W für >CH- steht oder Y¹¹ mit B beziehungsweise D über eine Einfachbindung verknüpft ist.
Dabei bedeuten R¹² und R¹³ unabhängig voneinander einen unverzweigten oder verzweigten Alkylrest mit 1 bis 15 Kohlenstoffatomen oder bilden zusammen eine Einheit -(CH₂)ₚ- mit p = 2, 3, 4, 5 oder 6, wobei eine, zwei oder drei dieser CH₂-Gruppen mit wenigstens einem unverzweigten oder verzweigten Alkylrest mit 1 bis 8 Kohlenstoffatomen substituiert sein können; vorzugsweise haben R¹² und R¹³ entweder die gleiche Bedeutung, sind also z.B. beide Ethyl oder n-Propyl, oder bilden zusammen eine wie oben definerte Einheit -(CH₂)ₚ-, die gegebenenfalls mit Alkylresten substituiert ist. Besonders bevorzugt stehen R¹² und R¹³ zusammen für -CH₂-CH₂-CH₂- oder -CH₂-C(CH₃)₂-CH₂-.
R¹⁴ bedeutet Aryl, Aralkyl, Alkyl, OH, Cl, Br, O-Aryl, O-Aralkyl oder O-Alkyl (Alkoxy), wobei die Aryl-, Aralkyl- und Alkylreste wie oben definiert sind. R¹⁴ ist vorzugsweise OH oder ein unsubstituierter geradkettiger oder verzweigter gesättigter O-Alkylrest (O-Alkanyl beziehungsweise Alkoxy) mit 1 bis 4 Kohlenstoffatomen, insbesondere O-Methyl, O-Ethyl, O-(n-Propyl), O-(iso-Propyl), O-(n-Butyl) oder O-(tert.-Butyl), oder ein O-Aralkylrest, insbesondere O-Benzyl.
R¹⁵ bedeutet Aryl, Aralkyl oder einen halogenierten oder unsubstituierten Alkylrest mit 1 bis 15 Kohlenstoffatomen, wobei in diesem Alkylrest auch eine oder mehrere CH₂-Gruppen jeweils unabhängig voneinander durch -C=C-, -CH=CH-, -O-, -CO-O- oder -O-CO- so ersetzt sein können, daß O-Atome nicht direkt miteinander verknüpft sind. R¹⁵ ist bevorzugt Aryl, insbesondere Phenyl oder 4-Toluyl, oder ein gesättigter unsubstituierter oder (per-)halogenierter Alkylrest mit 1 bis 4 Kohlenstoffatomen, insbesondere Methyl, Ethyl, n-Propyl, n-Butyl, tert.-Butyl, CF₃, n-C₄F₉ und t-C₄F₉.
R¹⁶ und R¹⁷ bedeuten H oder einen unverzweigten oder verzweigten Alkylrest mit 1 bis 15 Kohlenstoffatomen oder bilden zusammen eine Einheit -(CH₂)ₚ- mit p = 2, 3, 4, 5 oder 6, wobei eine, zwei oder drei dieser CH₂-Gruppen mit wenigstens einem unverzweigten oder verzweigten Alkylrest mit 1 bis 8 Kohlenstoffatomen substituiert sein können. Sie sind bevorzugt beide H oder beide ein gesättigter Alkylrest mit 1 bis 4 Kohlenstoffatomen, insbesondere Methyl, Ethyl, n-Propyl, iso-Propyl, n-Butyl oder tert.-Butyl, oder bilden gemeinsam eine cyclische Verbrückung, insbesondere -CH₂-CH₂-CH₂- oder -CH₂-C(CH₃)₂-CH₂-.
R¹⁸, R¹⁹ und R²⁰ bedeuten unabhängig voneinander einen unverzweigten oder verzweigten Alkylrest mit 1 bis 15 Kohlenstoffatomen. Vorzugsweise sind sie unabhängig voneinander gesättigte Alkylreste mit 1 bis 4 Kohlenstoffatomen, insbesondere Methyl, Ethyl, n-Propyl, iso-Propyl, n-Butyl oder tert.-Butyl. In besonders bevorzugten Ausführungsformen bedeuten R¹⁸, R¹⁹ und R²⁰ alle zugleich Methyl.
Kat⁺ ist ein Alkalimetallkation, d.h. Li⁺, Na⁺, K⁺, Rb⁺ oder Cs⁺, oder ein quartäres Ammoniumkation, zum Beispiel NH₄⁺ oder N(Alkyl)₄⁺, wobei Alkyl wie oben definert ist und die vier Alkylreste gleich oder verschieden sind. Bevorzugt ist Kat⁺ Na⁺, K⁺, NH₄⁺ oder ein Tetramethyl- oder Tetra-n-butylammomium-Kation
X⁻ ist ein schwach koordinierendes Anion, d.h. ein an das Schwefel-Kation nur relativ schwach bindender Ligand. Bevorzugt ist X⁻ BF₄⁻, CF₃SO₃⁻, C₄F₉SO₃⁻, PF₆⁻, SbF₆⁻ oder AsF₆⁻.

Unter den Substituenten Y¹¹, die über eine Einfachbindung verknüpft sind, sind besonders bevorzugt -H, -F, -Cl, -Br, -I, -OH, -CO₂H (d.h. -CO-R¹⁴ mit R¹⁴ = OH), -C(=S⁺R¹²)(-SR¹³)X⁻ und B(OR¹⁶)(OR¹⁷), insbesondere -Br, -OH, -C(=S⁺R¹²)(-SR¹³)X⁻, -B(OR¹⁶)(OR¹⁷), -BF₃⁻Kat⁺ und -Si(OR¹⁸)(OR¹⁹)(OR²⁰).

Wenn W direkt verbunden ist mit 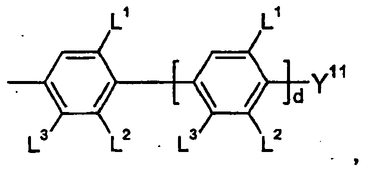 wobei d 0 oder 1 ist, ist Y¹¹ vorzugsweise
-H, -I, -OH, -SH, -CO₂R¹⁴, -OSO₂R¹⁵, -C(=S⁺R¹²)(SR¹³)X⁻, -B(OR¹⁶)(OR¹⁷), -BF₃⁻Kat⁺, -Si(OR¹⁸)(OR¹⁹)(OR²⁰) oder Alkyl, wobei Alkyl einen halogenierten oder unsubstituierten Alkylrest mit 1 bis 15 C-Atomen bedeutet, in welchem eine oder mehrere CH₂-Gruppen jeweils unabhängig voneinander durch -C≡C-, -CH=CH-, -O-, -CO-, -CO-O- oder -O-CO- so ersetzt sind, daß O-Atome nicht direkt miteinander verknüpft sind und Alkyl nicht für Alkoxy steht.

Bevorzugte erfindungsgemäße Verbindungen der Formel I sind solche, die durch eine der folgenden Formeln wiedergegeben werden:

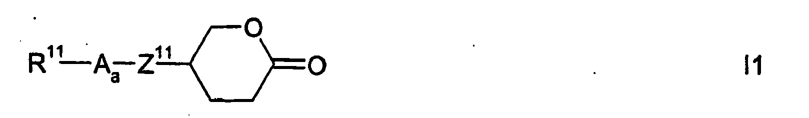

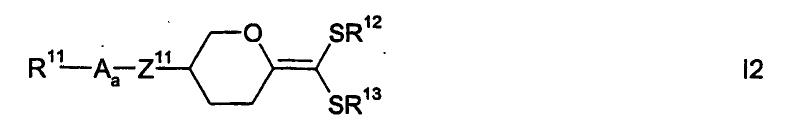

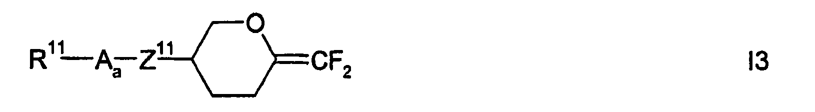

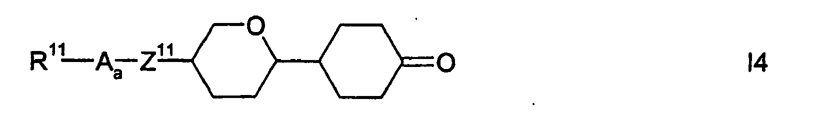

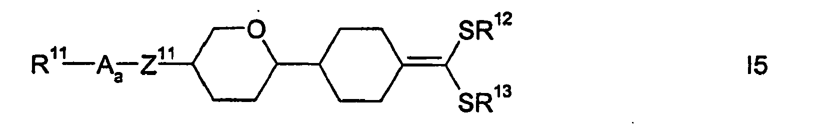

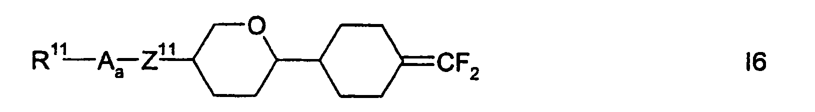

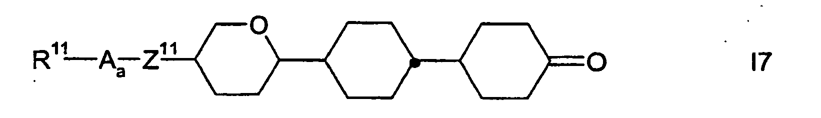

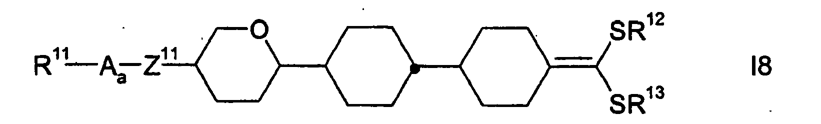

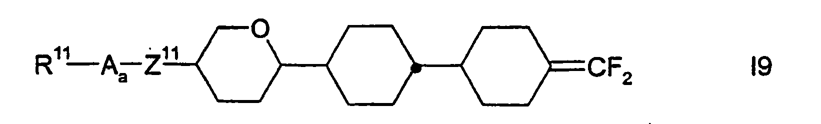

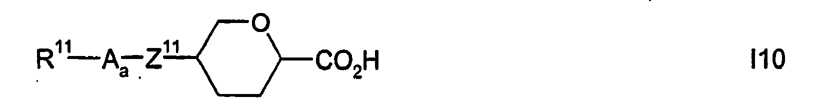

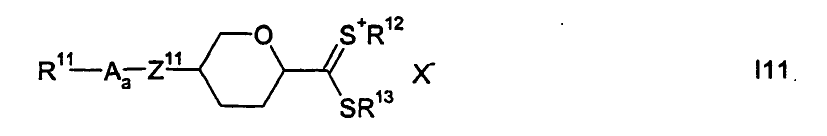

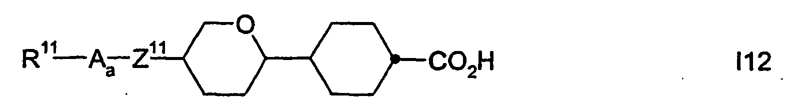

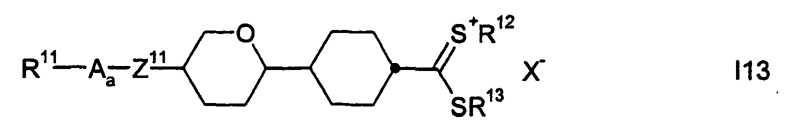

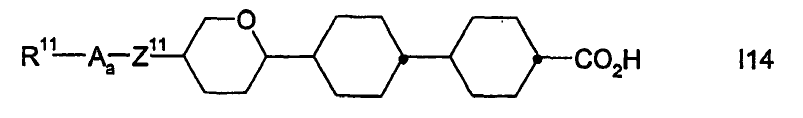

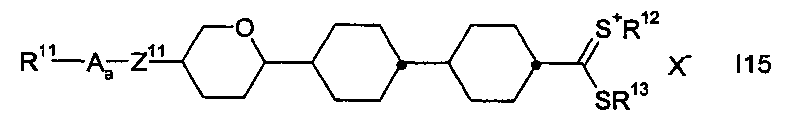

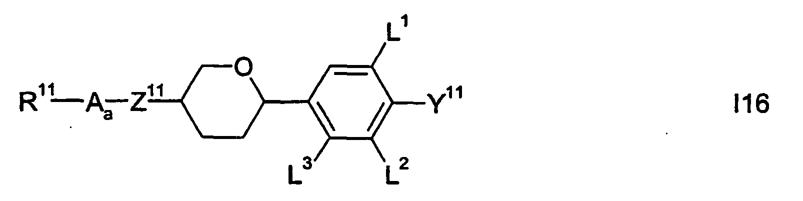

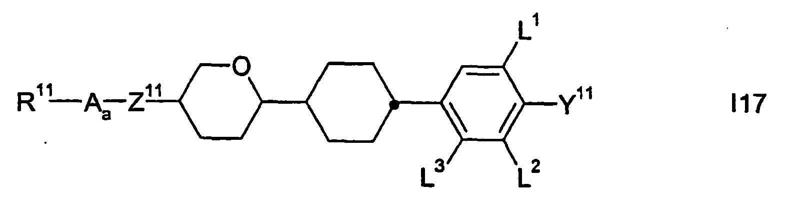

Dabei haben R¹¹, A, a, Z¹¹, Y¹¹, L¹, L², L³, R¹², R¹³ und X⁻ die oben angegebenen Bedeutungen.

Besonders bevorzugte Verbindungen der Formel I sind:

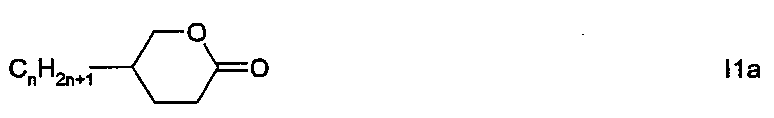

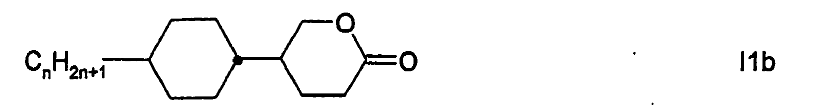

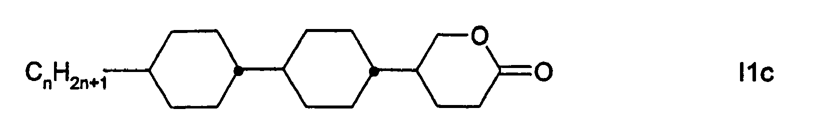

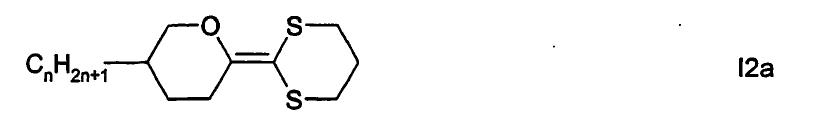

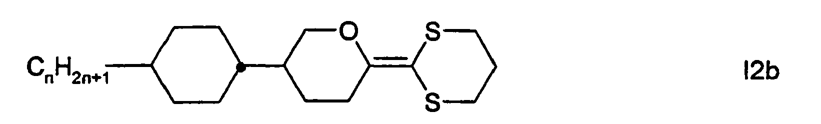

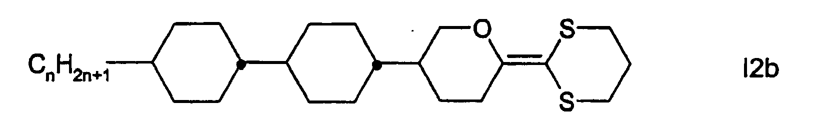

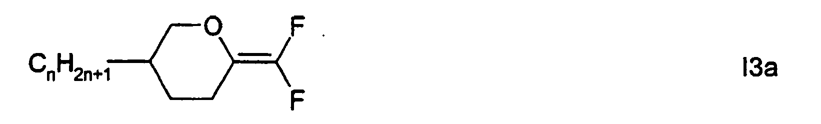

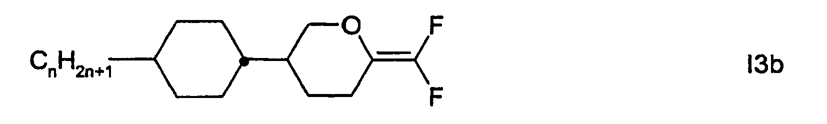

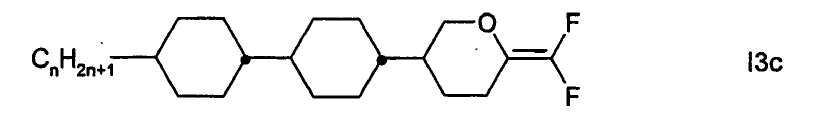

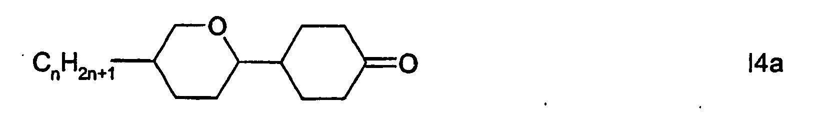

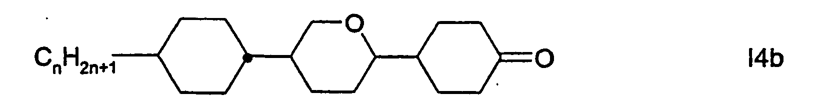

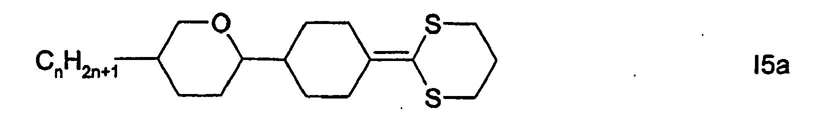

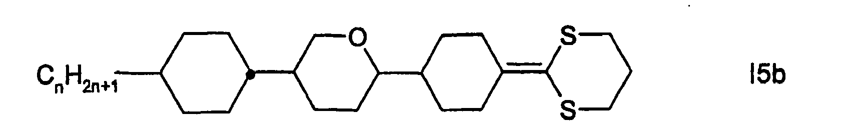

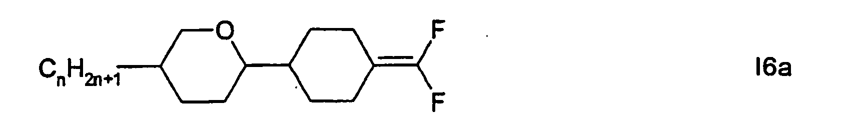

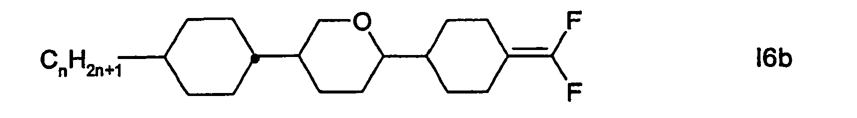

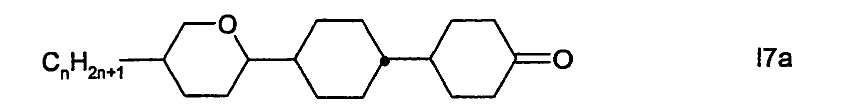

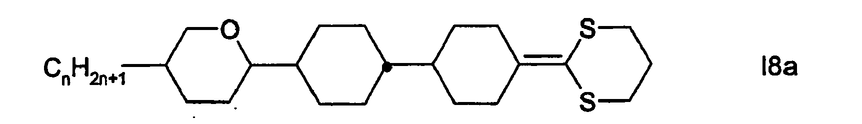

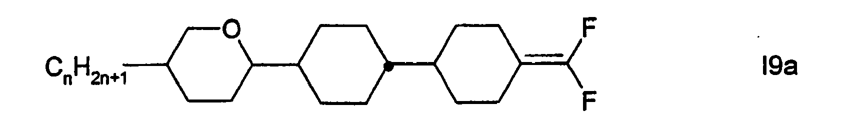

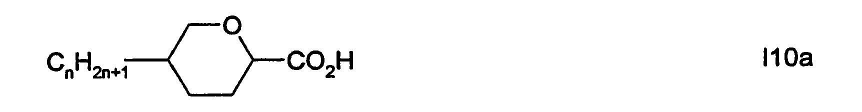

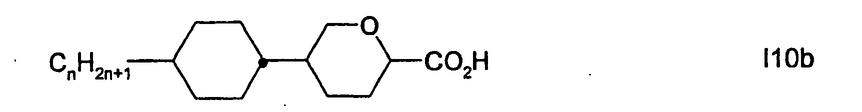

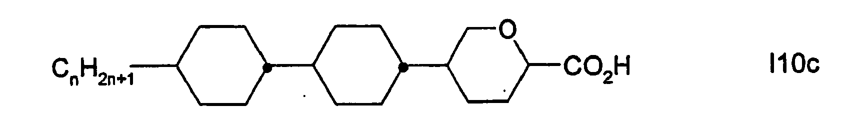

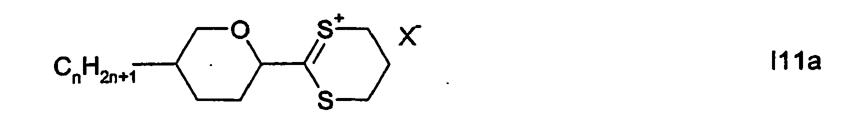

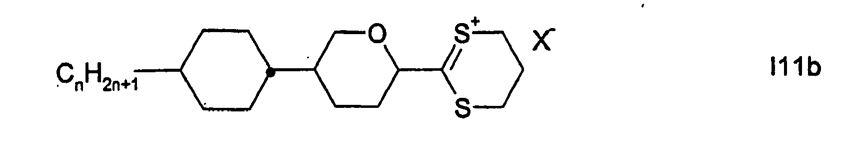

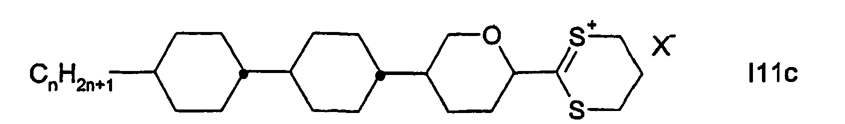

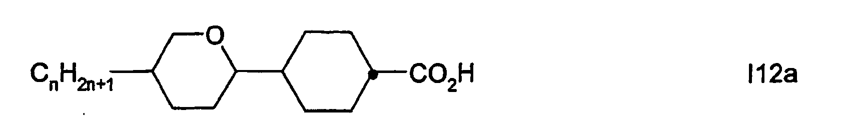

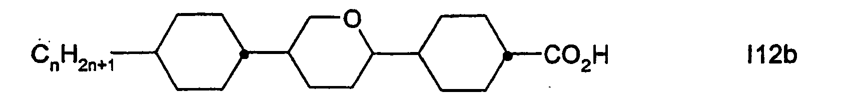

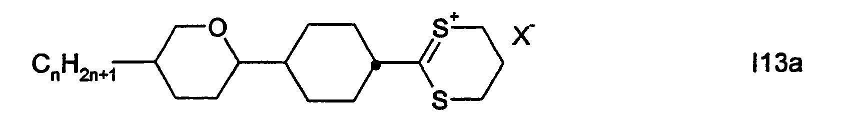

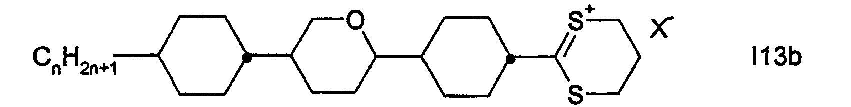

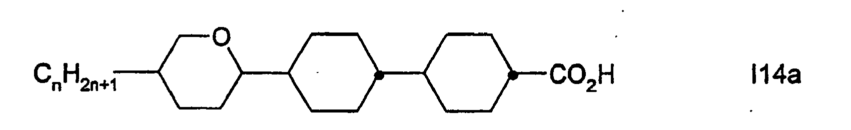

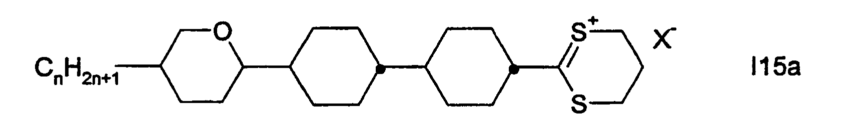

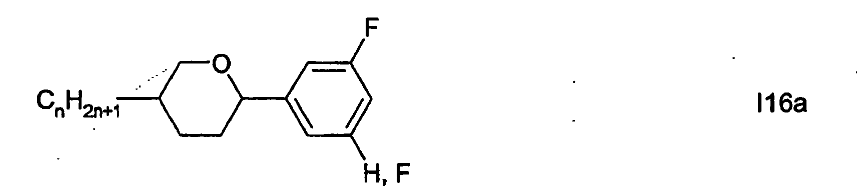

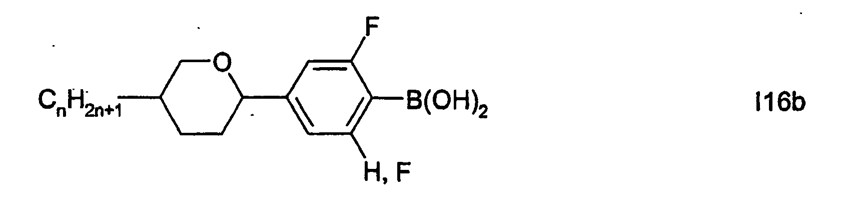

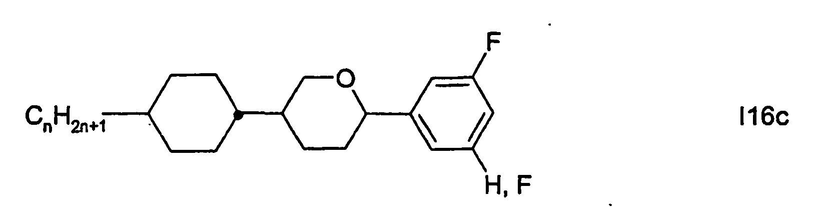

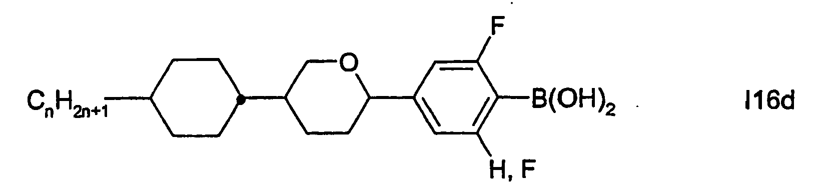

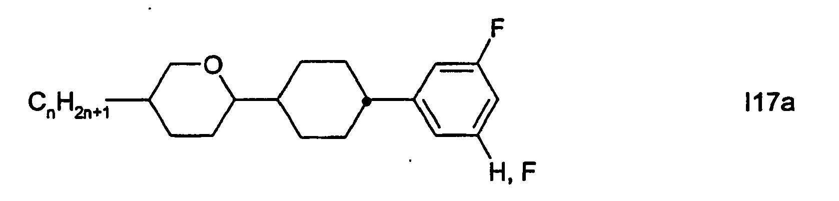

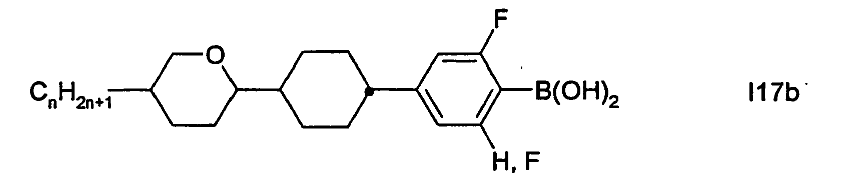

Dabei ist n eine ganze Zahl von 1 bis 7, insbesondere 1, 2, 3, 4 oder 5. CₙH₂ₙ₊₁ ist verzweigt oder vorzugsweise geradkettig.

Die erfindungsgemäßen Verbindungen der Formel I können nach an sich bekannten Methoden dargestellt werden, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Bevorzugt werden erfindungsgemäße Verbindungen der Formel I nach einem der folgenden erfindungsgemäßen Verfahren hergestellt:

In einem ersten Verfahren sind Verbindungen der Formel IA zugänglich 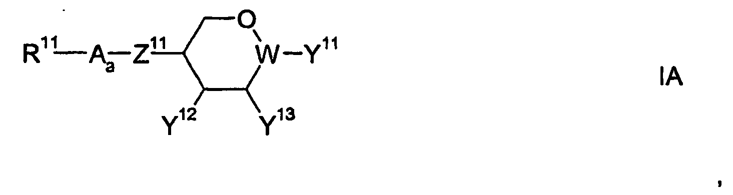 worin
W >C= bedeutet;
Y¹¹ =O, =C(SR¹²)(SR¹³) oder =CF₂ bedeutet; und
R¹¹, A, a, Z¹¹, Y¹², Y¹³, R¹² und R¹³ wie oben für Formel I definiert sind; wobei das Verfahren dadurch gekennzeichnet ist, daß
eine Verbindung der Formel II 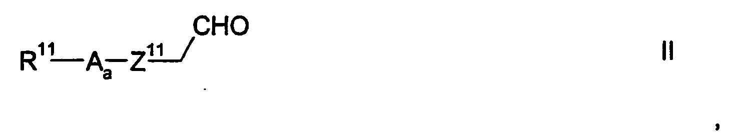 worin R¹¹, A, a und Z¹¹ wie oben für Formel IA definiert sind,
in einem Reaktionsschritt (A1)
(A1) in Gegenwart einer Base mit einer Verbindung der Formel III 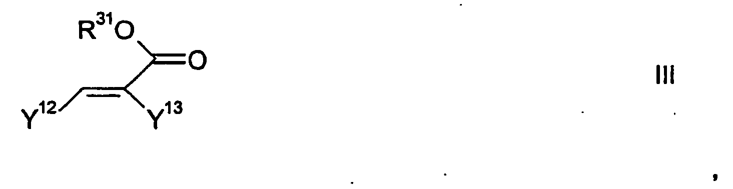 worin Y¹² und Y¹³ wie oben für Formel IA definiert sind und R³¹ ein Alkylrest mit 1 bis 15 Kohlenstoffatomen bedeutet, zu einer Verbindung der Formel IV 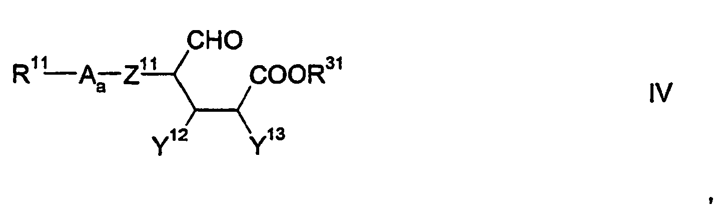 worin R¹¹, A, a, Z¹¹, Y¹² und Y¹³ wie oben für Formel IA definiert sind und R³¹ wie oben für Formel III definiert ist,
   umgesetzt wird;
   und anschließend in einem Reaktionsschritt (A2)
(A2) die Verbindung der Formel IV unter Reduktion der Aldehyd-Funktion und unter Lacton-Bildung in die Verbindung IA1 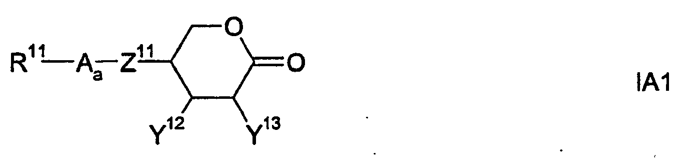 überführt wird;
   und gegebenenfalls in einem Reaktionsschritt (A3)
(A3) die Verbindung der Formel IA1 durch Umsetzung mit CF₂Br₂ in Gegenwart von P(N(R²¹)₂)₃, P(N(R²¹)₂)₂(OR²²) oder P(N(R²¹)₂)(OR²²)₂, wobei R²¹ und R²² unabhängig voneinander ein Alkylrest mit 1 bis 15 Kohlenstoffatomen ist, insbesondere Methyl oder Ethyl bedeutet, in die Verbindung IA2 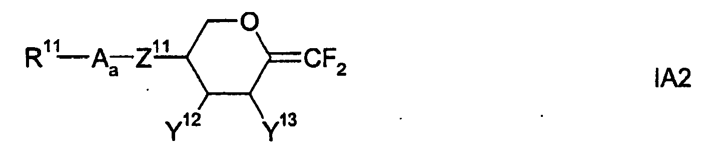 überführt wird;
   oder gegebenenfalls in einem Reaktionsschritt (A3')
(A3') die Verbindung der Formel IA1 durch Umsetzung mit CHG(SR¹²)(SR¹³), worin G P(OCH₂R²³)₃, wobei R²³ ein perfluorierter Alkylrest mit 1 bis 5 Kohlenstoffatomen, insbesondere CF₃, CF₂CF₃ oder C₄F₉ ist, oder Si(CH₃)₃ oder Si(CH₂CH₃)₃ bedeutet und R¹² und R¹³ wie oben für Formel IA definiert sind, in Gegenwart einer starken Base in die Verbindung IA3 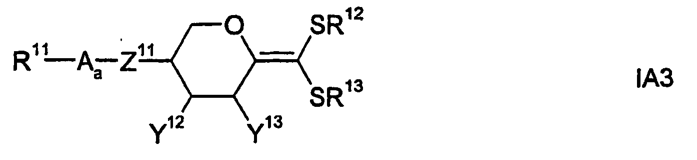 überführt wird.

Die Verbindungen der Formeln II und III sind literaturbekannt oder unter Anwendung beziehungsweise Abwandlung von im Stand der Technik bekannten Syntheseverfahren leicht zugänglich. Der Reaktionsschritt (A1) wird im allgemeinen bei Temperaturen oberhalb Raumtemperatur und vorzugsweise bei Rückflußtemperatur des Lösungsmittels durchgeführt. Als Lösungsmittel ist jedes Solvens geeignet, das die Reaktionspartner in für den Fortgang der Umsetzung ausreichendem Maße löst; vorzugsweise wird Acetonitril oder Propionitril verwendet. Als Base eignen sich solche Reagenzien, die mit der Verbindung der Formel II ein intermediäres Enamin bilden, insbesondere Dialkylamine und Trialkylsilyldialkylamine, zum Beispiel Trimethylsilyldiethylamin, welches dann mit der Verbindung der Formel III abreagiert. Die Reduktion der Aldehyd-Funktion der Verbindung der Formel IV zu einer Hydroxy-Funktion in Schritt (A2) erfolgt mit selektiv wirksamen Reduktionsmitteln, die im Stand der Technik bekannt sind, z.B. Natriumborhydrid. Die den Reaktionsschritt (A2) abschließende Lactonisierung zur Verbindung IA1 kann beispielsweise mit katalytischen Mengen einer Säure, etwa Toluolsulfonsäure, bewirkt werden.

Ausgehend von dem erfindungsgemäßen Lacton der Formel IA1 können, falls erwünscht, weitere erfindungsgemäße Verbindungen der Formeln IA2 oder IA3 in den Reaktionsschritten (A3) beziehungsweise (A3') dargestellt werden: Die Umsetzung von Verbindung IA1 zu Verbindung IA2 erfolgt mit Hilfe von CF₂Br₂ in Gegenwart von P(N(R²¹)₂)₃, P(N(R²¹)₂)₂(OR²²) oder P(N(R²¹)₂)(OR²²)₂, wobei R²¹ und R²² unabhängig voneinander ein Alkylrest mit 1 bis 15 Kohlenstoffatomen ist, insbesondere Methyl oder Ethyl bedeutet, in einem geeigneten Lösungsmittel, z.B. THF und/oder Dioxan, vorzugsweise bei Raumtemperatur. Reaktionsschritt (A3') wird in Gegenwart einer starken Base, vorzugsweise einer Lithium-organischen Base, wie n-Butyllithium, oder Kalium-tert.-butylat, bevorzugt in etherischen Lösungsmitteln wie THF bei Temperaturen zwischen -80 °C und Raumtemperatur ausgeführt.

In einem weiteren erfindungsgemäßen Verfahren sind Verbindungen der Formel IB zugänglich 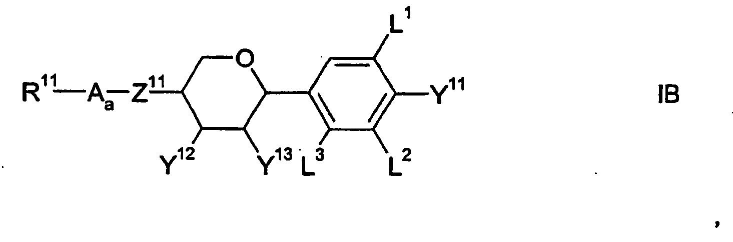 worin
Y¹¹ -H, -F, -Cl, -Br, -I, -CN, -OH oder -B(OR¹⁶)(OR¹⁷) bedeutet und R¹¹, A, a, Z¹¹, Y¹², Y¹³, L¹, L², L³, R¹⁶, R¹⁷ wie oben für Formel I definiert sind, und das dadurch gekennzeichnet ist, daß in einem Reaktionsschritt (B1)
(B1) eine Verbindung der Formel IA1 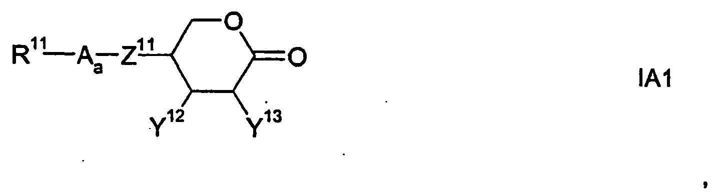 worin R¹¹, A, a, Z¹¹, Y¹² und Y¹³ wie oben für Formel IB definiert sind, mit einer Verbindung der Formel V 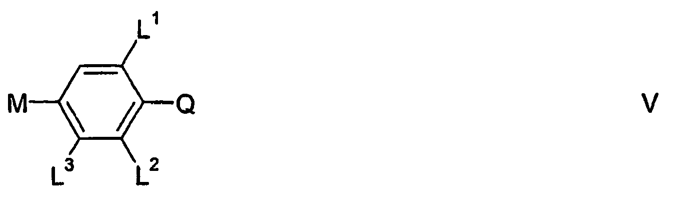 worin L¹, L² und L³ wie oben für Formel IB definiert sind, M Li, Cl-Mg, Br-Mg oder I-Mg bedeutet und Q H, F, Cl, Br, I oder CN bedeutet, unter Bildung der Verbindung der Formel IB1 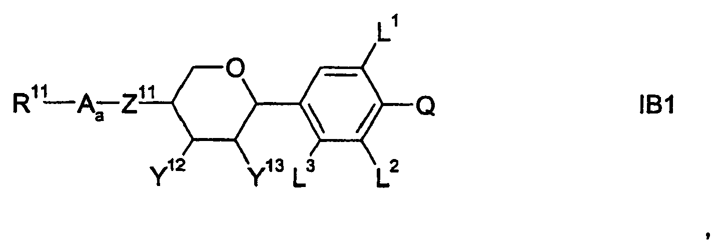 worin R¹¹, A, a, Z¹¹, Y¹², Y¹³, L¹, L² und L³ wie für Formel IB definiert sind und Q wie für Formel V definiert ist, umgesetzt wird; und gegebenenfalls in einem Reaktionsschritt (B2)
(B2) die Verbindung der Formel IB1, worin Q Br bedeutet, in Gegenwart einer Alkyl-Lithium-Base mit B(OR¹⁶)(OR¹⁷)(OR²⁴), wobei R¹⁶, R¹⁷ und R²⁴ einen unverzweigten oder verzweigten Alkylrest mit 1 bis 15 Kohlenstoffatomen bedeuten, oder mit HB(OR¹⁶)(OR¹⁷), wobei R¹⁶ und R¹⁷ einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können, zu der Verbindung der Formel IB2 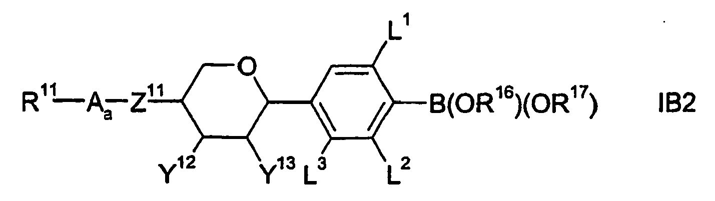 umgesetzt wird; und gegebenenfalls in einem Reaktionsschritt (B3)
(B3) die Verbindung IB2 durch Umsetzung mit einer wäßrigen Säure in die Verbindung IB3 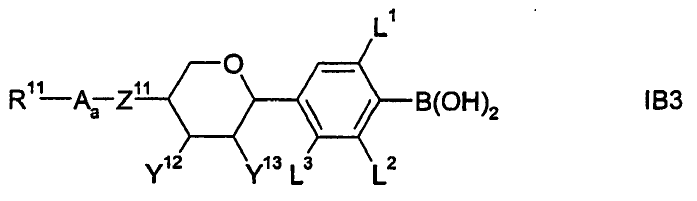 überführt wird;
   und/oder gegebenenfalls in einem Reaktionsschritt (B4)
(B4) die Verbindung IB2 oder die Verbindung IB3 durch Umsetzung mit Wasserstoffperoxid in alkalischer oder saurer Lösung, zum Beispiel in wäßriger Natronlauge oder Essigsäure, zu der Verbindung IB4 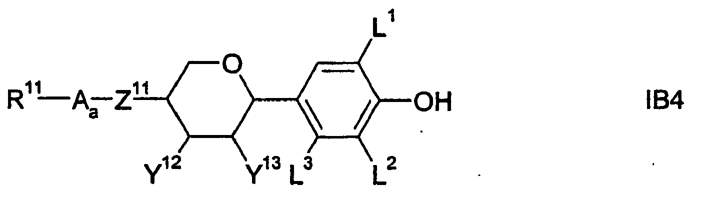 umgesetzt wird.

Die in Reaktionsschritt (B1) verwendete Verbindung der Formel V ist in an sich bekannter Weise aus der korrespondierenden halogenierten Verbindung durch Metall-Halogen-Austausch mittels einer Lithium-organischen Base oder nach Grignard zum Beispiel durch Umsetzung mit Magnesium zugänglich. Vorzugsweise bedeutet M in Formel V Lithium. Der Reaktionsschritt (B1) erfolgt bevorzugt in einem etherischen Lösungsmittel, z.B. Diethylether, bei Temperaturen zwischen -80 °C und Raumtemperatur.

Die Reduktion der nach der Addition der Verbindung V an die Carbonylfunktion der Verbindung der Formel IA1 intermediär gebildeten Hydroxy-Funktion zur Verbindung der Formel IB1 erfolgt mit einem geeigneten Reduktionsmittel, z.B. Trialkylsilan und Bortrifluorid-Etherat.

Bei der im optional durchführbaren Reaktionsschritt (B2) verwendeten Alkyl-Lithium-Base handelt es sich bevorzugt um Methyllithium oder n-Butyllithium, vorzugsweise in einem etherischen Lösungsmittel, z.B. THF, oder in Hexan. Nach erfolgtem Lithium-Halogen-Austausch (vorzugsweise bei Temperaturen zwischen -80 °C und Raumtemperatur) wird mit einem geeigneten Borsäurederivat, z.B. Trialkylborat, unter Bildung der Verbindung der Formel IB2 umgesetzt. Aus dieser kann, falls erwünscht, in Reaktionsschritt (B3) die korrespondierende Borsäure der Formel IB3 mit wäßriger Säure, z.B. 2N Salzsäure, freigesetzt werden. In dem optional durchführbaren Reaktionsschritt (B4) kann - ausgehend von IB2 oder IB3 - mit Wasserstoffperoxid in alkalischer Lösung, z.B. 30 % H₂O₂ in Natronlauge, oder saurer Lösung, z.B. in Essigsäure, das entsprechende Phenol der Formel IB4 hergestellt werden.

In einem weiteren erfindungsgemäßen Verfahren sind erfindungsgemäße Verbindungen der Formel IC erhältlich: 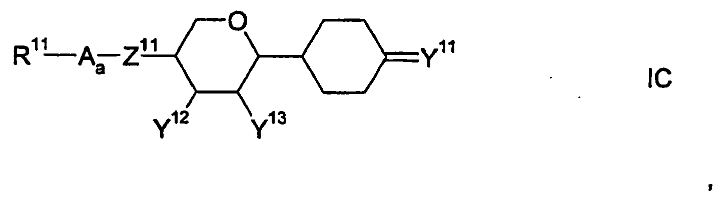 worin
Y¹¹ =O, =C(SR¹²)(SR¹³) oder =CF₂ bedeutet und
R¹¹, A, a, Z¹¹, Y¹², Y¹³, R¹² und R¹³ wie oben für Formel I definiert sind, und das dadurch gekennzeichnet ist, daß in einem Reaktionsschritt (C1)
(C1) die Verbindung der Formel IB4 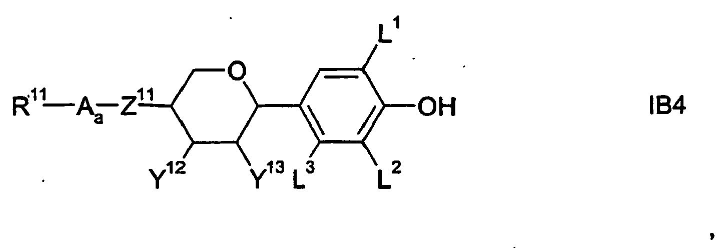 worin R¹¹, A, a, Z¹¹, Y¹² und Y¹³ wie oben für Formel IC definiert sind und
   L¹, L² und L³ H bedeuten,
   mit Wasserstoff in Gegenwart eines Übergangsmetall-Katalysators in die Verbindung IC1 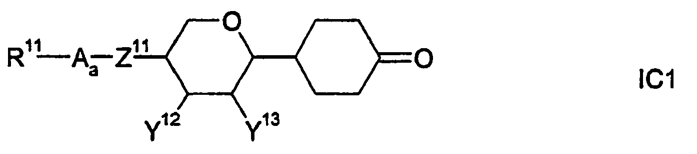 überführt wird (d.h. in die Verbindung IC mit Y¹¹ gleich =O);
   und gegebenenfalls in einem Reaktionsschritt (C2)
(C2) die Verbindung IC1 durch Umsetzung mit CF₂Br₂ in Gegenwart von P(N(R²¹)₂)₃, P(N(R²¹)₂)₂(OR²²) oder P(N(R²¹)₂)(OR²²)₂, wobei R²¹ und R²² unabhängig voneinander ein Alkylrest mit 1 bis 15 Kohlenstoffatomen sind und insbesondere Methyl oder Ethyl bedeuten, in die Verbindung IC2 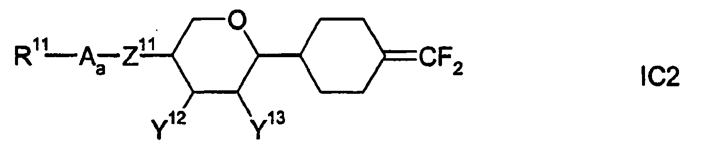 überführt wird (d.h. die Verbindung IC mit Y¹¹ gleich =CF₂);
   oder gegebenenfalls in einem Reaktionsschritt (C2')
(C2') die Verbindung der Formel IC1 durch Umsetzung mit
   CHG(SR¹²)(SR¹³), worin G P(OCH₂R²³)₃, wobei R²³ ein perfluorierter Alkylrest mit 1 bis 5 Kohlenstoffatomen, insbesondere CF₃, CF₂CF₃ oder C₄F₉ ist, oder Si(CH₃)₃ oder Si(CH₂CH₃)₃ bedeutet und R¹² und R¹³ wie oben für Formel IC definiert sind, in Gegenwart einer starken Base in die Verbindung IC3 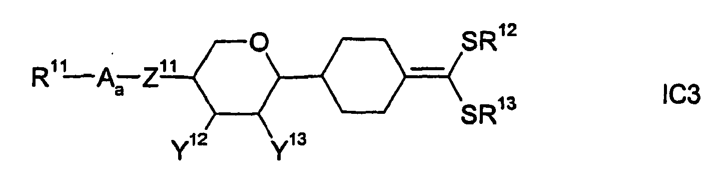 überführt wird (d.h. die Verbindung IC mit Y¹¹ gleich =C(SR¹²)(SR¹³)).

Bei dem in Reaktionsschritt (C1) verwendeten Übergangsmetall-Katalysator handelt es sich um einen üblicherweise für Hydrierungsreaktionen verwendeten Katalysator, z.B. Palladium auf Kohlenstoff; es werden die für diese Art der Hydrierung üblichen Reaktionsbedingungen gewählt (siehe zum Beispiel J. March: Advanced Organic Chemistry; John Wiley & Sons, New York u.a., 3. Aufl., 1985, S. 700-702, 5-11). Die weiteren optionalen Reaktionsschritte (C2) beziehungsweise (C2') werden im wesentlichen unter den gleichen Bedingungen wie die Reaktionsschritte (A3) und (A3') oben ausgeführt.

In noch einem weiteren erfindungsgemäßen Verfahren werden erfindungsgemäße Verbindungen der Formel ID hergestellt: 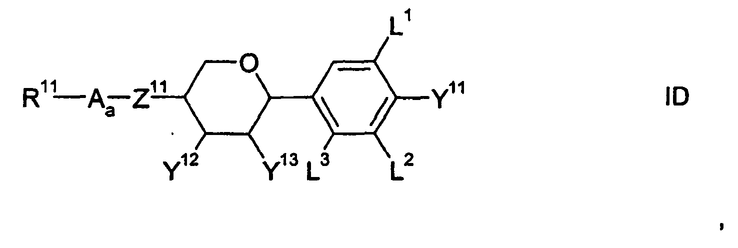 worin
Y¹¹ -CO₂H oder -C(=S⁺R¹²)(-SR¹³)X⁻ bedeutet und R¹¹, A, a, Z¹¹, Y¹², Y¹³, R¹², R¹³, L¹, L², L³ und X⁻ wie oben für Formel I definiert sind, und das dadurch gekennzeichnet ist, daß in einem Reaktionsschritt (D1)
(D1) eine Verbindung der Formel IB1 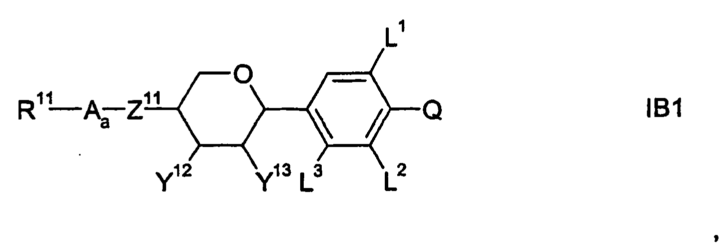 worin R¹¹, A, a, Z¹¹, Y¹², Y¹³, L¹, L² und L³ wie für Formel ID definiert sind und Q H oder Br bedeutet,
   zunächst mit einer metallorganischen Base und dann mit CO₂ zu der Verbindung ID1 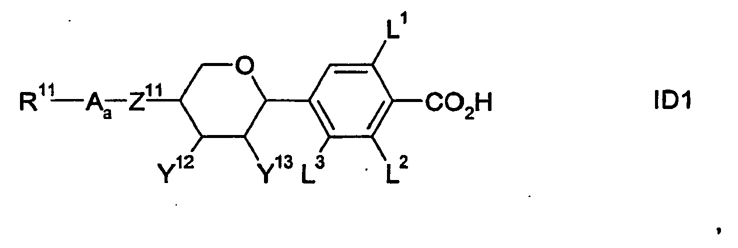 worin R¹¹, A, a, Z¹¹, Y¹², Y¹³, L¹, L² und L³ wie für Formel ID definiert sind, umgesetzt wird;
   und gegebenenfalls in einem Reaktionsschritt (D2)
(D2) die Verbindung ID1 in Gegenwart einer Säure HX mit HSR¹² und HSR¹³ oder mit HSR¹²R¹³SH in die Verbindung ID2 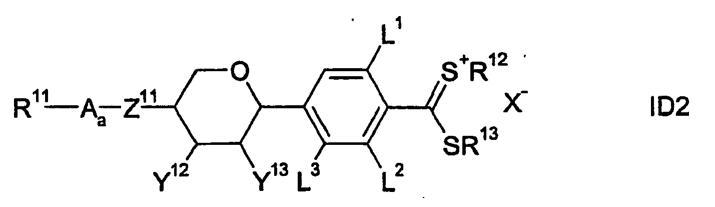 überführt wird.

Die in Reaktionsschritt (D1) verwendete metallorganische Base ist vorzugsweise eine Lithium-organische Base, z.B. n-Butyllithium in THF oder Hexan; die Metallierung erfolgt bevorzugt bei Temperaturen zwischen -40 °C und Raumtemperatur. Das zum Abfangen der intermediär gebildeten metallierten Verbindung verwendete CO₂ kann als gasförmiges Kohlendioxid eingeleitet oder bevorzugt in fester Form als Trockeneis zugesetzt werden. Nach wäßriger Aufarbeitung wird das gewünschte Benzoesäure-Derivat ID1 erhalten.

In dem optional durchführbaren Reaktionsschritt (D2) werden als Thiol-Reagenz vorzugsweise Dithiole, z.B. 1,3-Propanthiol oder 2,2-Dimethylpropan-1,3-dithiol, in Gegenwart einer Säure, z.B. Toluolsulfonsäure, verwendet.

In einem weiteren erfindungsgemäßen Verfahren sind weitere erfindungsgemäße Verbindungen der Formel IE zugänglich: 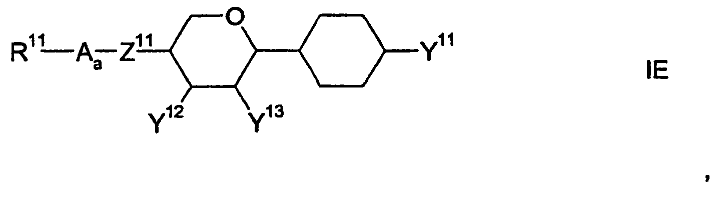 worin
Y¹¹ -CO₂H oder -C(=S⁺R¹²)(-SR¹³)X⁻ bedeutet und R¹¹, A, a, Z¹¹, Y¹², Y¹³, R¹² und R¹³ und X⁻ wie oben für Formel I definiert sind,
und das dadurch gekennzeichnet ist, daß in einem Reaktionsschritt (E1)
(E1) die Verbindung der Formel ID1 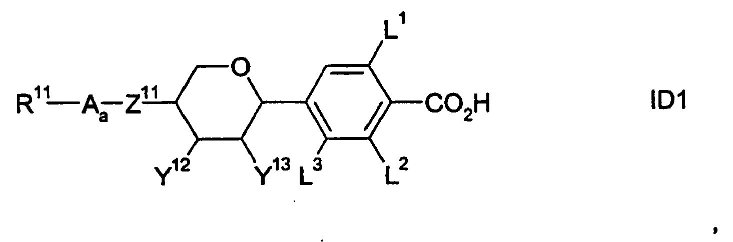 worin R¹¹, A, a, Z¹¹, Y¹² und Y¹³ wie oben für Formel IE definiert sind und L¹, L² und L³ H bedeuten,
   mit Wasserstoff in Gegenwart eines Übergangsmetall-Katalysators in die Verbindung IE1 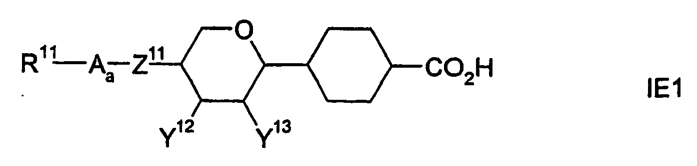 überführt wird;
   und gegebenenfalls in einem Reaktionsschritt (E2)
(E2) die Verbindung der Formel IE1 in Gegenwart einer Säure HX mit HSR¹² und HSR¹³ oder mit HSR¹²R¹³SH in die Verbindung IE2 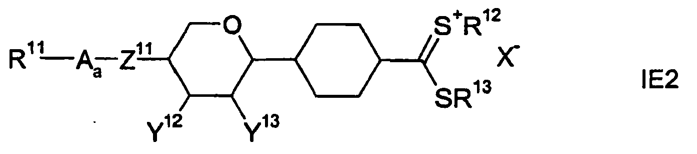 überführt wird.

Für Einzelheiten bei der Durchführung der Reaktionsschritte (E1) und (E2) wird auf die Reaktionsschritte (C1) beziehungsweise (D2) verwiesen.

Die Fachleute werden erkennen, daß durch entsprechende Anpassung beziehungsweise durch Wiederholung bestimmter Reaktionsschritte der erfindungsgemäßen Verfahren die Synthese weiterer erfindungsgemäßer Verbindungen erfolgen kann. Sie werden ferner mit Hilfe ihres Fachwissens in der Lage sein, die exakte Einstellung von Reaktionsparametem wie der genauen Menge der Reaktanten, der Reaktionszeit und -temperatur und der Auswahl und Quantität der Reagenzien und des Lösungsmittels nach den Notwendigkeiten der jeweiligen Umsetzung vorzunehmen.

Sofern die erfindungsgemäßen Verbindungen in Form optischer Isomeren auftreten können, etwa in Gestalt von Enantiomeren und/oder Diastereomeren - wenn sie z.B. einen chiralen Rest R¹¹ tragen -, so sind diese Verbindungen vom Umfang der Beschreibung und der Ansprüche mit umfaßt. Diese chiralen Verbindungen können als Gemisch der Isomeren, vorzugsweise als Racemat oder aber in isomerenreiner Form vorliegen. Die isomerenreinen oder isomerenangereicherten erfindungsgemäßen Verbindungen sind in an sich bekannter Weise durch übliche Trennverfahren, z.B. Chromatographie an chiralen Phasen oder fraktionierte Kristallisation gegebenenfalls in Gegenwart chiraler Reagenzien, oder durch gezielte asymmetrische Synthese zugänglich.

Die erfindungsgemäßen Verbindungen können als Ausgangsverbindungen für komplexe, einen Tetrahydropyranring enthaltende Moleküle eingesetzt werden. So sind beispielsweise ausgehend von Verbindungen der Formeln I2, I3, I5, I6, I8, I9, I11, I13 beziehungsweise I15 in Analogie zu an sich literaturbekannten Verfahren die entsprechenden Verbindungen erhältlich, in denen der endständige Rest (Y¹¹) durch eine -CF₂O-Aryl-Gruppe ersetzt ist. Derartig funktionalisierte Tetrahydropyran-Derivate weisen mesogene Eigenschaften auf.

Die vorliegende Erfindung wird durch die nachfolgenden Beispiele weiter veranschaulicht, ohne daß diese den Umfang der Erfindung beschränken sollen.

### Beispiele

Die in den Beispielen eingesetzten Ausgangsverbindungen, Reagenzien und Lösungsmittel sind - soweit nichts abweichendes angegeben ist - käuflich erhältlich. Die Charakterisierung der Produkte erfolgte mittels üblicher Analysemethoden, unter anderem GC, HPLC, Massenspektrometrie, ¹H- und ¹⁹F-NMR-Spektroskopie, DSC und Polarisationsmikroskopie.

Die Herstellung der Verbindungen der Beispiele 1 bis 7 folgte dem nachstehenden Syntheseschema 1:

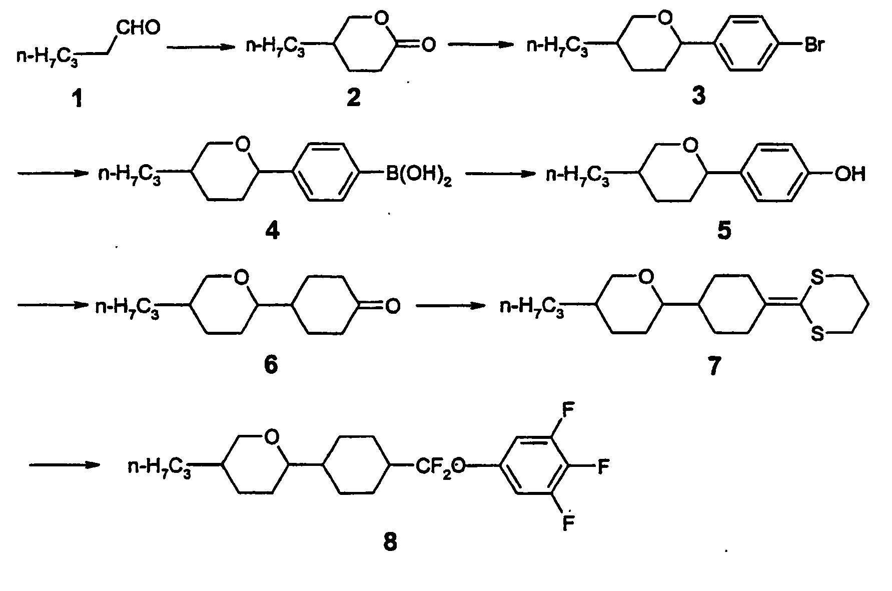

### Beispiel 1: Verbindung 2

Eine Mischung von 500 mmol Pent-1-al (1), 700 mmol Acrylsäuremethylester, 50 mmol Trimethylsilyldiethylamin und 300 ml Acetonitril wurde 18 h zum Sieden erhitzt. Man engte im Vakuum ein, wobei Acetonitril und Silane entfernt wurden. Danach wurden 2,5 mol Eisessig hinzugegeben und für 18 h bei 50 °C gerührt. Man neutralisierte durch Zugabe von 2N NaOH und verdünnte mit 3 l ges. NaCl-Lösung. Die organische Phase wurde abgetrennt, mit ges. NaCl-Lösung gewaschen und über Na₂SO₄ getrocknet. Man setzte 1 I Isopropanol und anschließend 250 mmol Natriumborhydrid zu und rührte 18 h. Die Mischung wurde wäßrig aufgearbeitet, die organische Phase zweimal mit ges. NaCl-Lösung gewaschen und mit Na₂SO₄ getrocknet. Der rohe Alkohol wurde mit 1 I Toluol und 1 g Toluolsulfonsäure bis zur Vollständigkeit der Reaktion am Wasserabscheider gekocht. Die Lösung wurde neutral gewaschen und anschließend durch Destillation gereinigt. Ausbeute des Lactons (2): 35 % einer intensiv nach Waldmeister riechenden Flüssigkeit. Siedepunkt: 137-138 °C (19 mbar).

### Beispiel 2: Verbindung 3

Eine Lösung von 207 mmol 1,4-Dibrombenzol in 250 ml Diethylether wurde bei -50 °C tropfenweise mit 207 mmol n-Butyllithiurn (15 % in Hexan) versetzt. Dann tropfte man eine Lösung von 170 mmol 2 in 50 ml Diethylether bei derselben Temperatur zu, rührte 30 min nach, ließ das Gemisch sich auf 0 °C erwärmen und arbeitete wäßrig auf. Das Rohprodukt (51 g) wurde in 400 ml Dichlormethan gelöst und bei -75 °C mit 400 mmol Triethylsilan versetzt. Man tropfte 400 mmol Bortrifluorid-Etherat zu, wobei die Temperatur unter -70 °C gehalten wurde. Danach ließ man die Temperatur auf -10 °C ansteigen, hydrolysierte mit gesättigter Natriumhydrogencartionat-Lösung und arbeitete wäßrig auf. Das Rohprodukt enthielt die trans/cis-Isomeren in einem Verhältnis von 9:1. Man kristallisierte aus Pentan bei -20 °C um; Ausbeute der Verbindung 3: 30,6 g, 61 %. Schmelzpunkt: 43 °C.

### Beispiel 3: Verbindung 4

73 mmol 3 wurden in 200 ml THF gelöst und auf -70 °C gekühlt. Man tropfte zuerst 73 mmol n-Butyllithium (15 % in Hexan) zu, gefolgt von 73 mmol Trimethylborat in 50 ml THF. Man ließ die Mischung sich auf -20 °C erwärmen, stellte durch Zugabe von 2N HCl auf pH 2 ein und arbeitete wäßrig auf. Das Rohprodukt wurde mit heißem Heptan digeriert und bei 0 °C kristallisiert. Ausbeute der Verbindung 4: 13,1 g, 72 %.

### Beispiel 4: Verbindung 5

Eine Mischung von 60 mmol 4, 300 ml Toluol, 120 mmol NaOH, 50 ml Wasser und 30 ml 30% H₂O₂ wurde 2 h bei 45 °C gerührt. Die Mischung wurde mit 10% HCl auf pH 2 eingestellt und wäßrig aufgearbeitet. Das Rohprodukt wurde aus Heptan umkristallisiert. Ausbeute der Verbindung 5: 7,1 g, 52%.

### Beispiel 5: Verbindung 6

22 mmol 5 wurden in 100 ml Xylol in der Gegenwart von 1,5 g 5% Pd-C-Katalysators bei 5 bar und 130 °C für 27,5 h hydriert. Die Aufarbeitung erfolgte wie üblich. Ausbeute der Verbindung 6: 65%, farbloses Öl.

### Beispiel 6: Verbindung 7

Eine Lösung von 17 mmol 2-Trimethylsilyl-1,3-dithian in 75 ml THF wurde bei -70 °C mit 17 mmol n-Butyllithium (15% in Hexan) versetzt. Man ließ innerhalb von 4 h auf 0 °C kommen, kühlte dann wieder auf -70 °C und tropfte 17 mmol 6 in 25 ml THF zu, ließ auf Raumtemperatur kommen, rührte 18 h nach und arbeitete wäßrig auf. Das Rohprodukt wurde aus Heptan kristallisiert. Ausbeute der Verbindung 7: 40%, farblose Kristalle.

### Beispiel 7: Verbindung 8

Eine Lösung von 6,12 mmol 7 in 50 ml Dichlormethan wurde bei -20 °C tropfenweise mit 6,27 mmol Trifluormethansulfonsäure versetzt. Man ließ für 30 min auf Raumtemperatur kommen und kühlte dann auf -70 °C ab. Dann wurden zuerst eine Lösung 9,1 mmol 3,4,5-Trifluorphenol und 10,1 mmol Triethylamin in 20 ml Dichlormethan, 5 min später 31 mmol Triethylamin-Tris(hydrofluorid) zugegeben. Nach weiteren fünf Minuten gab man in kleinen Portionen eine Suspension von 31,5 mmol DBH (1,3-Dibrom-5,5-dimethylhydanthoin) zu und rührte eine Stunde bei -70 °C nach. Man ließ auf -10 °C kommen und goß die Reaktionsmischung in 400 ml eiskalte NaOH. Man arbeitete wäßrig auf und reinigte das Rohprodukt und reinigte durch Chromatographie an Kieselgel (Heptan/Toluol 3:2) und Kristallisation aus Pentan bei -70 °C. Ausbeute der Verbindung **8:** 42%, farblose Kristalle. Schmelzpunkt: 35 °C. Klärpunkt: 66.3 °C.

## Patentansprüche

1. Verbindung der allgemeinen Formel I 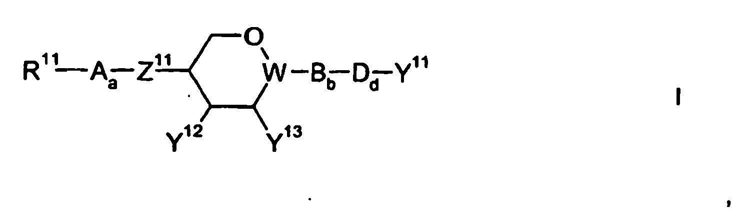 worin
R¹¹ H, F, Cl, Br, I, CN,
aromatische Kohlenwasserstoffreste mit vorzugsweise 6 bis 18 Kohlenstoffatomen, die gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂ oder C₁₋₁₅-Alkyl einfach oder mehrfach, gleich oder verschieden, substituiert sind, heterocyclische Reste, in denen wenigstens ein Ringatom ein Heteroatom aus der Gruppe O, S und N besteht, wobei der Rest gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂, Aryl oder C₁₋₁₅-Alkyl einfach oder mehrfach, gleich oder verschieden, substituiert sein kann, oder
einen halogenierten oder unsubstituierten Alkylrest mit 1 bis 15 Kohlenstoffatomen bedeutet, wobei in diesem Rest auch eine oder mehrere CH₂-Gruppen jeweils unabhängig voneinander durch -C=C-, -CH=CH-, -O-, -CO-, -CO-O- oder -O-CO- so ersetzt sein können, daß O-Atome nicht direkt miteinander verknüpft sind;
A für 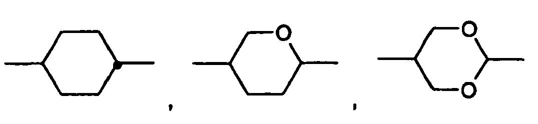 oder 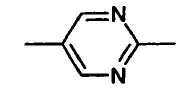 steht;
a 0, 1 oder 2 ist;
Z¹¹ eine Einfachbindung, -CH₂-CH₂-, -CF₂-CF₂-, -CF₂-CH₂-, -CH₂-CF₂-, -CH₂-O-, -O-CH₂-, -CF₂-O- oder -O-CF₂- darstellt;
W >CH- oder >C= bedeutet;
B und D unabhängig voneinander für 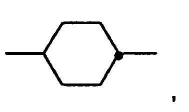 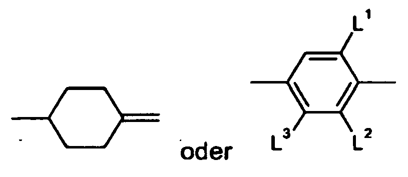 stehen;
b und d unabhängig voneinander 0 oder 1 sind;
Y¹¹ =O, =C(SR¹²)(SR¹³), =CF₂, -H, -F, -Cl, -Br, -I, -CN, -OH, -SH, -CO-R¹⁴, -OSO₂R¹⁵, -C(=S⁺R¹²)(-SR¹³)X⁻, -B(OR¹⁶)(OR¹⁷), -BF₃⁻Kat⁺, -Si(OR¹⁸)(OR¹⁹)(OR²⁰) oder Alkyl bedeutet, wobei Alkyl einen halogenierten oder unsubstituierten Alkylrest mit 1 bis 15 C-Atomen bedeutet, in welchem auch eine oder mehrere CH₂-Gruppen jeweils unabhängig voneinander durch -C≡C-, -CH=CH-, -O-, -CO-, -CO-O- oder -O-CO- so ersetzt sein können, daß O-Atome nicht direkt miteinander verknüpft sind;
Y¹² und Y¹³ unabhängig voneinander H oder Alkyl bedeuten, wobei Alkyl einen halogenierten oder unsubstituierten Alkylrest mit 1 bis 15 C-Atomen bedeutet, in welchem auch eine oder mehrere CH₂-Gruppen jeweils unabhängig voneinander durch -C≡C-, -CH=CH-, -O-, -CO-, -CO-O- oder -O-CO- so ersetzt sein können, daß O-Atome nicht direkt miteinander verknüpft sind;
L¹, L² und L³ unabhängig voneinander H oder F bedeuten;
R¹² und R¹³ unabhängig voneinander einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können;
R¹⁴ OH, O-Aryl, O-Alkyl-Aryl, O-C₁₋₁₅-Alkyl. Cl, Br, Aryl, Aryl-C₁₋₁₅-Alkyl oder C₁₋₁₅-Alkyl,
worin Aryl aromatische Kohlenwasserstoffreste mit vorzugsweise 6 bis 18 Kohlenstoffatomen, die gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂ oder C₁₋₁₅-Alkyl einfach oder mehrfach, gleich oder verschieden, substituiert sind bedeutet,
bedeutet,;
R¹⁵ Aryl, Aryl-C₁₋₁₅-Alkyl oder einen halogenierten oder unsubstituierten Alkylrest mit 1 bis 15 Kohlenstoffatomen bedeutet, wobei in diesem Alkylrest auch eine oder mehrere CH₂-Gruppen jeweils unabhängig voneinander durch -C≡C-, -CH=CH-, -O-, -CO-, -CO-O- oder -O-CO- so ersetzt sein können, daß O-Atome nicht direkt miteinander verknüpft sind, worin Aryl aromatische Kohlenwasserstoffreste mit vorzugsweise 6 bis 18 Kohlenstoffatomen, die gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂ oder C₁₋₁₅-Alkyl einfach oder mehrfach, gleich oder verschieden, substituiert sind bedeutet;
R¹⁶ und R¹⁷ H oder einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können;
R¹⁸, R¹⁹ und R²¹ unabhängig voneinander einen unverzweigten oder verzweigten Alkylrest mit 1 bis 15 Kohlenstoffatomen bedeuten;
Kat⁺ ein Alkalimetallkation oder ein quartäres Ammoniumkation ist;
und
X ein schwach koordinierendes Anion ist;
mit der Maßgabe,
daß W >CH- bedeutet, wenn b+d ≠ 0;
daß Y¹¹ nicht =O, =C(SR¹²)(SR¹³) oder =CF₂ bedeutet, wenn Y¹¹ mit B oder 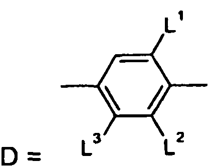 verbunden ist;
daß Y¹¹ -H, -I, -SH, -CO₂R¹⁴, -OSO₂R¹⁵, -C(=S⁺R¹²)(SR¹³)X⁻, -B(OR¹⁶)(OR¹⁷), -BF₃⁻Kat⁺, -Si(OR¹⁸)(OR¹⁹)(OR²⁰) oder Alkyl bedeutet, wobei Alkyl einen halogenierten oder unsubstituierten Alkylrest mit 1 bis 15 C-Atomen bedeutet, in welchem eine oder mehrere CH₂-Gruppen jeweils unabhängig voneinander durch -C≡C-, -CH=CH-, -O-, -CO-, -CO-O- oder -O-CO- so ersetzt sind, daß O-Atome nicht direkt miteinander verknüpft sind und Alkyl nicht für Alkoxy steht, wenn W direkt verbunden ist mit 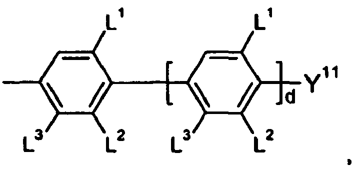 wobei d 0 oder 1 ist;
daß B nicht für 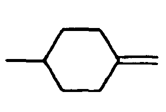 steht, wenn d = 1; und
daß A gleiche oder verschiedene Bedeutungen annehmen kann, wenn a 2 ist.

2. Verbindung nach Anspruch 1, **dadurch gekennzeichnet, daß**
A für 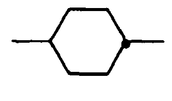 steht.

3. Verbindung nach Anspruch 1, **dadurch gekennzeichnet, daß**
a 0 ist.

4. Verbindung nach irgendeinem der Ansprüche 1 bis 3, **dadurch gekennzeichnet, daß**
Y¹² und Y¹³ H bedeuten.

5. Verbindung nach irgendeinem der Ansprüche 1 bis 4, **dadurch gekennzeichnet, daß**
Z¹¹ eine Einfachbindung, -CF₂O- oder -OCF₂- darstellt.

6. Verbindung nach irgendeinem der Ansprüche 1 bis 5, **dadurch gekennzeichnet, daß**
R¹¹ einen unverzweigten halogenierten oder unsubstituierten Alkylrest mit 1 bis 7 Kohlenstoffatomen bedeutet.

7. Verbindung nach irgendeinem der Ansprüche 1 bis 6, **dadurch gekennzeichnet, daß**
Y¹¹ =O, =C(SR¹²)(SR¹³) oder =CF₂ bedeutet.

8. Verbindung nach irgendeinem der Ansprüche 1 bis 6, **dadurch gekennzeichnet, daß**
Y¹¹ -H, -F, -Cl, -Br, -I, -OH, -CO₂H, -C(=S⁺R¹²)(-SR¹³)X⁻, -B(OR¹⁶)(OR¹⁷), -BF₃-Kat⁺ oder -Si(OR¹⁸)(OR¹⁹)(OR²⁰) bedeutet.

9. Verbindung nach irgendeinem der Ansprüche 1 bis 6 und 8, **dadurch gekennzeichnet, daß**
X⁻ BF₄⁻, CF₃SO₃⁻, C₄F₉SO₃⁻, PF₆⁻, SbF₆⁻ oder AsF₆⁻ bedeutet.

10. Verbindung nach irgendeinem der Ansprüche 1 bis 9, **dadurch gekennzeichnet, daß**
b 0 ist und d 0 ist.

11. Verbindung nach irgendeinem der Ansprüche 1 bis 9, **dadurch gekennzeichnet, daß**
b 1 ist und d 0 ist.

12. Verbindung nach irgendeinem der Ansprüche 1 bis 9, **dadurch gekennzeichnet, daß**
b 1 ist und d 1 ist.

13. Verfahren zur Herstellung einer Verbindung der Formel IA 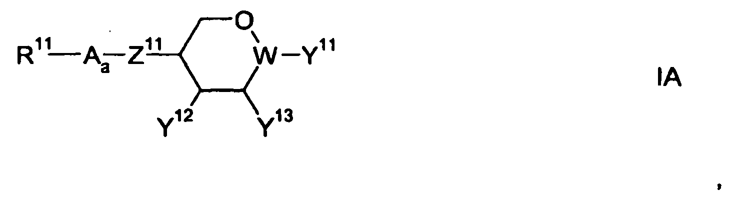 worin
R¹¹ H, F, Cl, Br, I, CN,
aromatische Kohlenwasserstoffreste mit vorzugsweise 6 bis 18 Kohlenstoffatomen, die gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂ oder C₁₋₁₅-Alkyl einfach oder mehrfach, gleich oder verschieden, substituiert sind,
heterocyclische Reste, in denen wenigstens ein Ringatom ein Heteroatom aus der Gruppe O, S und N besteht, wobei der Rest gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂, Aryl oder C₁₋₁₅-Alkyl einfach oder mehrfach, gleich oder verschieden, substituiert sein kann, oder
C₁₋₁₅-Alkyl bedeutet;
A für 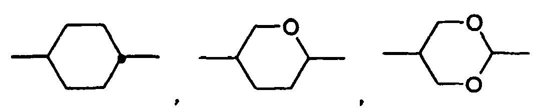 oder 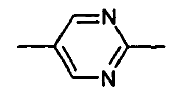 steht;
a 0, 1 oder 2 ist, wobei A gleiche oder verschiedene Bedeutungen annehmen kann, wenn a 2 ist;
Z¹¹ eine Einfachbindung, -CH₂-CH₂-, -CF₂-CF₂-. -CF₂-CH₂-, -CH₂-CF₂-, -CH₂-O-, -O-CH₂-, -CF₂-O- oder -O-CF₂- darstellt;
W >C= bedeutet;
Y¹¹ =O, =C(SR¹²)(SR¹³) oder =CF₂ bedeutet;
Y¹² und Y¹³ unabhängig voneinander H oder C₁₋₁₅-Alkyl bedeuten; und
R¹² und R¹³ unabhängig voneinander einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können;
**dadurch gekennzeichnet, daß**
eine Verbindung der Formel II 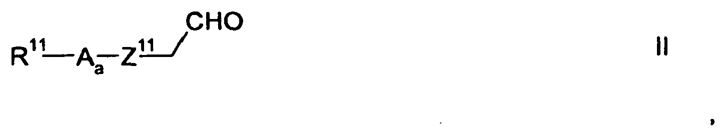 worin R¹¹, A, a und Z¹¹ wie oben für Formel IA definiert sind,
in einem Reaktionsschritt (A1)
(A1) in Gegenwart einer Base mit einer Verbindung der Formel III 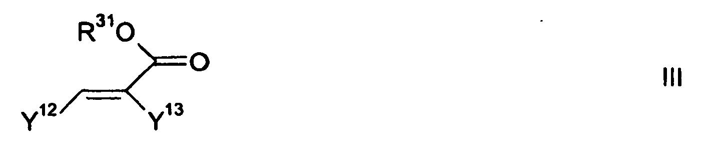 worin Y¹² und Y¹³ wie oben für Formel IA definiert sind und R³¹ ein Alkylrest mit 1 bis 15 Kohlenstoffatomen bedeutet, zu einer Verbindung der Formel IV 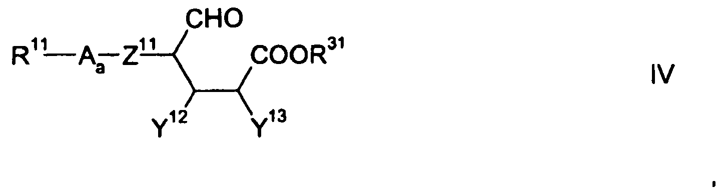 worin R¹¹, A, a, Z¹¹, Y¹² und Y¹³ wie oben für Formel IA definiert sind und R³¹ wie oben für Formel III definiert ist,
umgesetzt wird;
und anschließend in einem Reaktionsschritt (A2)
(A2) die Verbindung der Formel IV in die Verbindung IA1 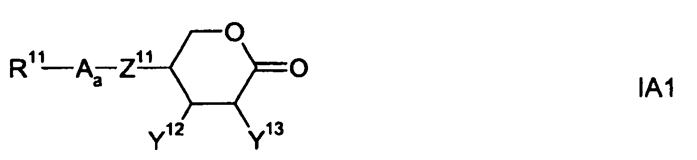 überführt wird;
und gegebenenfalls in einem Reaktionsschritt (A3)
(A3) die Verbindung der Formel IA1 durch Umsetzung mit CF₂Br₂ in Gegenwart von P(N(R²¹)₂)₃, P(N(R²¹)₂)₂(OR²²) oder
P(N(R²¹)₂)(OR²²)₂, wobei R²¹ und R²² unabhängig voneinander ein Alkylrest mit 1 bis 15 Kohlenstoffatomen sind, in die Verbindung IA2 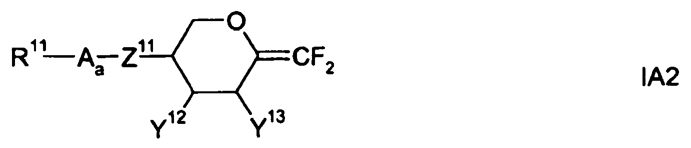 überführt wird;
oder gegebenenfalls in einem Reaktionsschritt (A3')
(A3') die Verbindung der Formel IA1 durch Umsetzung mit CHG(SR¹²)(SR¹³), worin G P(OCH₂R²³)₃, wobei R²³ ein perfluorierter Alkylrest mit 1 bis 5 Kohlenstoffatomen ist, oder Si(CH₃)₃ oder Si(CH₂CH₃)₃ bedeutet und R¹² und R¹³ wie oben für Formel IA definiert sind, in Gegenwart einer starken Base in die Verbindung IA3 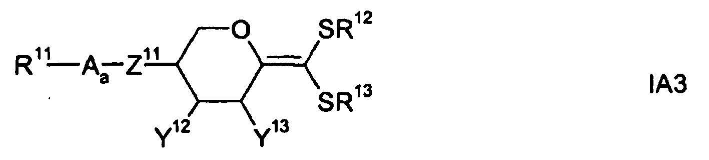
überführt wird.

14. Verfahren zur Herstellung einer Verbindung der Formel IB 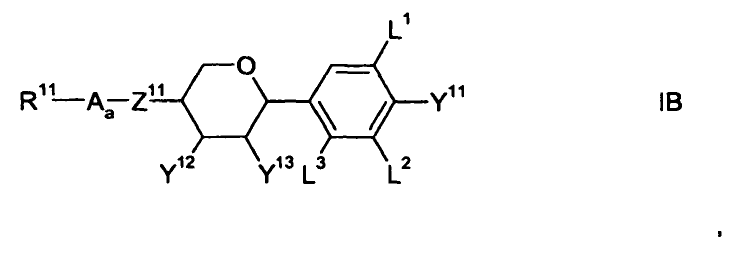 worin
R¹¹ H, F, Cl, Br, I, CN,
aromatische Kohlenwasserstoffreste mit vorzugsweise 6 bis 18 Kohlenstoffatomen, die gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂ oder C₁₋₁₅-Alkyl einfach oder mehrfach, gleich oder verschieden, substituiert sind,
heterocyclische Reste, in denen wenigstens ein Ringatom ein Heteroatom aus der Gruppe O, S und N besteht, wobei der Rest gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂, Aryl oder C₁₋₁₅-Alkyl einfach oder mehrfach, gleich oder verschieden, substituiert sein kann, oder
C₁₋₁₅-Alkyl bedeutet;
A für 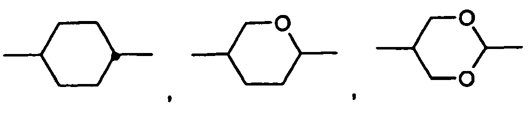 oder 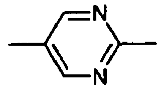 steht;
a 0, 1 oder 2 ist, wobei A gleiche oder verschiedene Bedeutungen annehmen kann, wenn a 2 ist;
Z¹¹ eine Einfachbindung, -CH₂-CH₂-, -CF₂-CF₂-, -CF₂-CH₂-, -CH₂-CF₂-, -CH₂-O-, -O-CH₂-, -CF₂-O- oder -O-CF₂- darstellt;
Y¹¹ -H, -F, -Cl, -Br, -I, -CN, -OH oder -B(OR¹⁶)(OR¹⁷) bedeutet;
Y¹² und Y¹³ unabhängig voneinander H oder C₁₋₁₅-Alkyl bedeuten;
L¹, L² und L³ unabhängig voneinander H oder F bedeuten; und
R¹⁶ und R¹⁷ unabhängig voneinander H oder einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können;
**dadurch gekennzeichnet, daß**
in einem Reaktionsschritt (B1)
(B1) eine Verbindung der Formel IA1 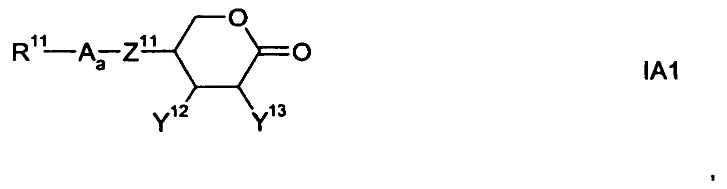 worin R¹¹, A, a, Z¹¹, Y¹² und Y¹³ wie oben für Formel IB definiert sind, mit einer Verbindung der Formel V 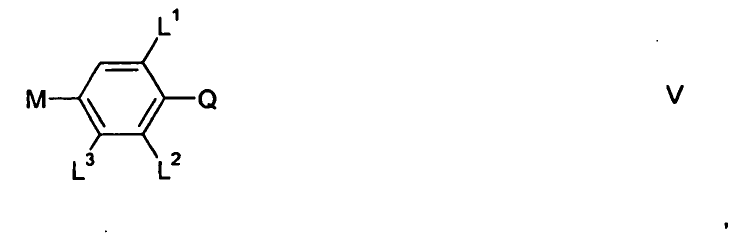 worin L¹, L² und L³ wie oben für Formel IB definiert sind, M Li, Cl-Mg, Br-Mg oder I-Mg bedeutet und Q H, F, Cl, Br, I oder CN bedeutet,
unter Bildung der Verbindung der Formel IB1 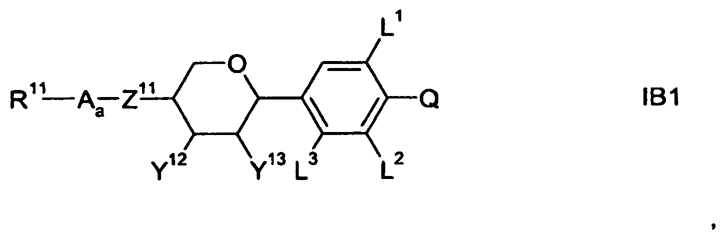 worin R¹¹, A, a, Z¹¹, Y¹², Y¹³, L¹, L² und L³ wie für Formel IB definiert sind und Q wie für Formel V definiert ist,
umgesetzt wird;
und gegebenenfalls in einem Reaktionsschritt (B2)
(B2) die Verbindung der Formel IB1, worin Q Br bedeutet, in Gegenwart einer Alkyl-Lithium-Base mit B(OR¹⁶)(OR¹⁷)(OR²⁴), wobei R¹⁶, R¹⁷ und R²⁴ einen unverzweigten oder verzweigten Alkylrest mit 1 bis 15 Kohlenstoffatomen bedeuten, oder mit HB(OR¹⁶)(OR¹⁷), wobei R¹⁶ und R¹⁷ einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können, zu der Verbindung der Formel IB2 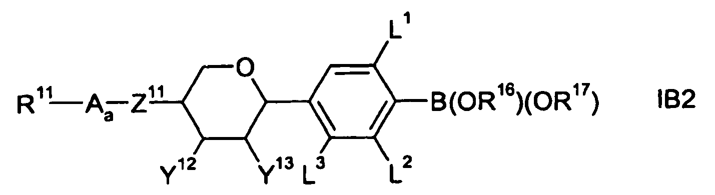 umgesetzt wird;
und gegebenenfalls in einem Reaktionsschritt (B3)
(B3) die Verbindung IB2 durch Umsetzung mit einer wäßrigen Säure in die Verbindung IB3 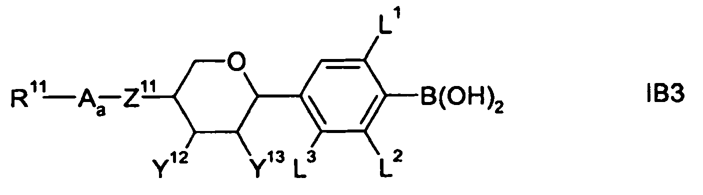 überführt wird;
und/oder gegebenenfalls in einem Reaktionsschritt (B4)
(B4) die Verbindung IB2 oder die Verbindung IB3 durch Umsetzung mit Wasserstoffperoxid in alkalischer oder saurer Lösung zu der Verbindung IB4 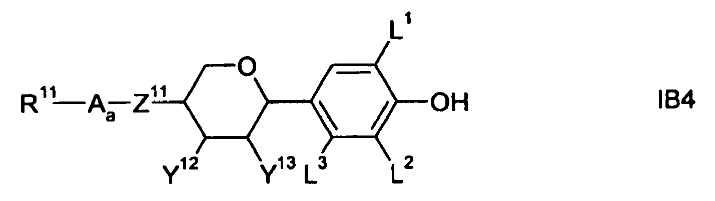
umgesetzt wird.

15. Verfahren zur Herstellung einer Verbindung der allgemeinen Formel IC 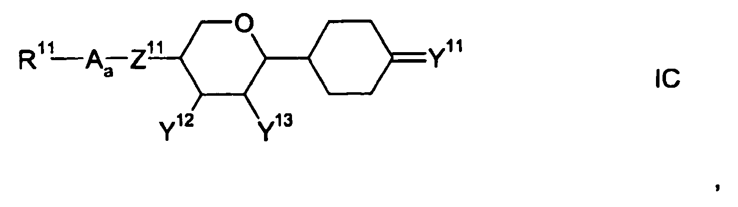 worin
R¹¹ H, F, Cl, Br, I, CN,
aromatische Kohlenwasserstoffreste mit vorzugsweise 6 bis 18 Kohlenstoffatomen, die gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂ oder C₁₋₁₅-Alkyl einfach oder mehrfach, gleich oder verschieden, substituiert sind,
heterocyclische Reste, in denen wenigstens ein Ringatom ein Heteroatom aus der Gruppe O, S und N besteht, wobei der Rest gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂, Aryl oder C₁₋₁₅-Alkyl einfach oder mehrfach, gleich oder verschieden, substituiert sein kann, oder
C₁₋₁₅-Alkyl bedeutet;
A für 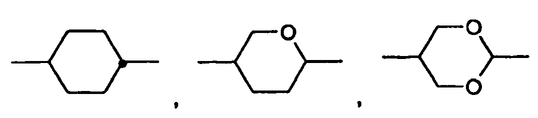 oder 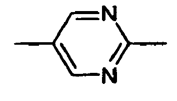 steht;
a 0, 1 oder 2 ist, wobei A gleiche oder verschiedene Bedeutungen annehmen kann, wenn a 2 ist;
Z¹¹ eine Einfachbindung, -CH₂-CH₂-, -CF₂-CF₂-, -CF₂-CH₂-, -CH₂-CF₂-, -CH₂-O-, -O-CH₂-, -CF₂-O- oder -O-CF₂- darstellt;
Y¹¹ =O, =C(SR¹²)(SR¹³) oder =CF₂ bedeutet;
Y¹² und Y¹³ unabhängig voneinander H oder C₁₋₁₅-Alkyl bedeuten; und
R¹² und R¹³ unabhängig voneinander einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können;
**dadurch gekennzeichnet, daß** in einem Reaktionsschritt (C1)
(C1) die Verbindung der Formel IB4 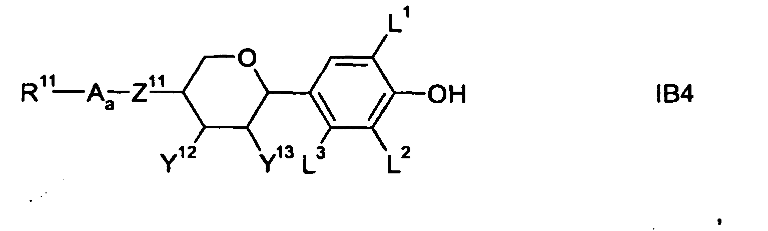 worin R¹¹, A, a, Z¹¹, Y¹² und Y¹³ wie oben für Formel IC definiert sind und L¹, L² und L³ H bedeuten,
mit Wasserstoff in Gegenwart eines Übergangsmetall-Katalysators in die Verbindung IC1 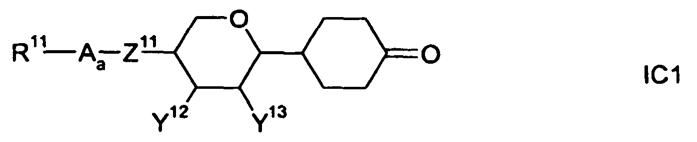 überführt wird;
und gegebenenfalls in einem Reaktionsschritt (C2)
(C2) die Verbindung IC1 durch Umsetzung mit CF₂Br₂ in Gegenwart von P(N(R²¹)₂)₃, P(N(R²¹)₂)₂(OR²²) oder P(N(R²¹)₂)(OR²²)₂, wobei R²¹ und R²² unabhängig voneinander ein Alkylrest mit 1 bis 15 Kohlenstoffatomen sind, in die Verbindung IC2 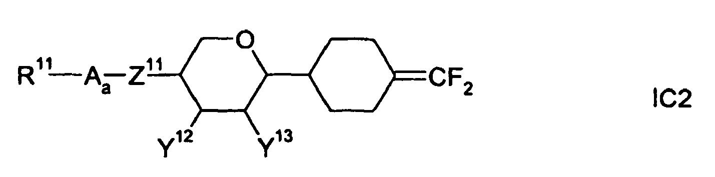 überführt wird;
oder gegebenenfalls in einem Reaktionsschritt (C2')
(C2') die Verbindung der Formel IC1 durch Umsetzung mit CHG(SR¹²)(SR¹³), worin G P(OCH₂R²³)₃, wobei R²³ ein perfluorierter Alkylrest mit 1 bis 5 Kohlenstoffatomen ist, oder Si(CH₃)₃ oder Si(CH₂CH₃)₃ bedeutet und R¹² und R¹³ wie oben für Formel IC definiert sind, in Gegenwart einer starken Base in die Verbindung IC3 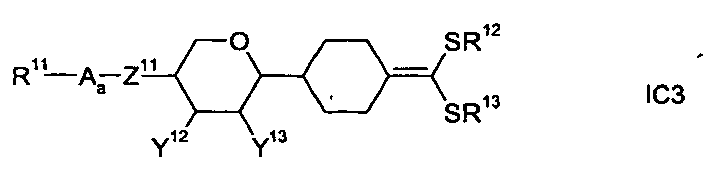
überführt wird.

16. Verfahren zur Herstellung einer Verbindung der Formel ID 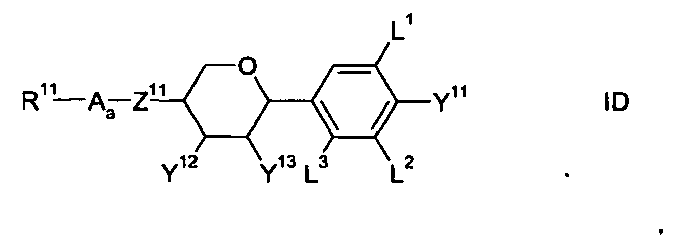 worin
R¹¹ H, F, Cl, Br, I, CN,
aromatische Kohlenwasserstoffreste mit vorzugsweise 6 bis 18 Kohlenstoffatomen, die gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂ oder C₁₋₁₅-Alkyl einfach oder mehrfach, gleich oder verschieden, substituiert sind,
heterocyclische Reste, in denen wenigstens ein Ringatom ein
Heteroatom aus der Gruppe O, S und N besteht, wobei der Rest gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂, Aryl oder C₁₋₁₅-Alkyl einfach oder mehrfach, gleich oder verschieden, substituiert sein kann, oder
C₁₋₁₅-Alkyl bedeutet;
A für 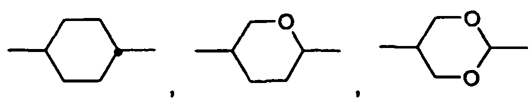 oder 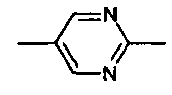 steht;
a 0, 1 oder 2 ist, wobei A gleiche oder verschiedene Bedeutungen annehmen kann, wenn a 2 ist;
Z¹¹ eine Einfachbindung, -CH₂-CH₂-, -CF₂-CF₂-, -CF₂-CH₂-, -CH₂-CF₂-, -CH₂-O-, -O-CH₂-, -CF₂-O- oder -O-CF₂- darstellt;
Y¹¹ -CO₂H oder -C(=S⁺R¹²)(-SR¹³)X⁻ bedeutet;
Y¹² und Y¹³ unabhängig voneinander H oder C₁₋₁₅-Alkyl bedeuten;
L¹, L² und L³ unabhängig voneinander H oder F bedeuten;
R¹² und R¹³ unabhängig voneinander einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können; und
X⁻ ein schwach koordinierendes Anion ist;
**dadurch gekennzeichnet, daß** in einem Reaktionsschritt (D1)
(D1) eine Verbindung der Formel IB1 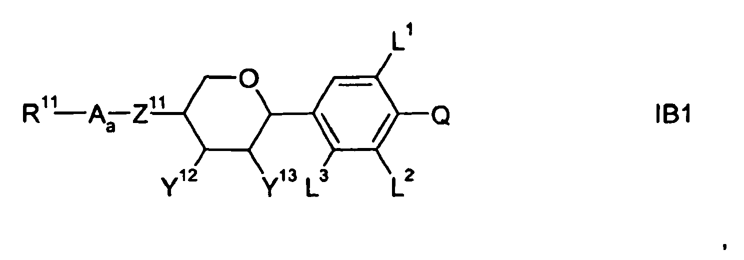 worin R¹¹, A, a, Z¹¹, Y¹², Y¹³, L¹, L² und L³ wie für Formel ID definiert sind und Q H oder Br bedeutet,
mit einer metallorganischen Base und CO₂ zu der Verbindung ID1 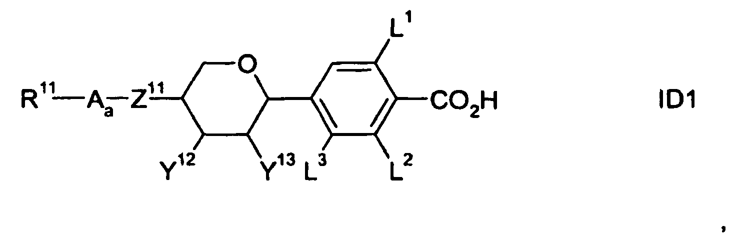 worin R¹¹, A, a, Z¹¹, Y¹², Y¹³, L¹, L² und L³ wie für Formel ID definiert sind, umgesetzt wird;
und gegebenenfalls in einem Reaktionsschritt (D2)
(D2) die Verbindung ID1 in Gegenwart einer Säure HX mit HSR¹² und HSR¹³ oder mit HSR¹²R¹³SH in die Verbindung ID2 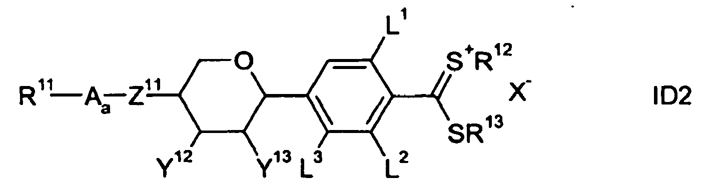
überführt wird.

17. Verfahren zur Herstellung einer Verbindung der Formel IE 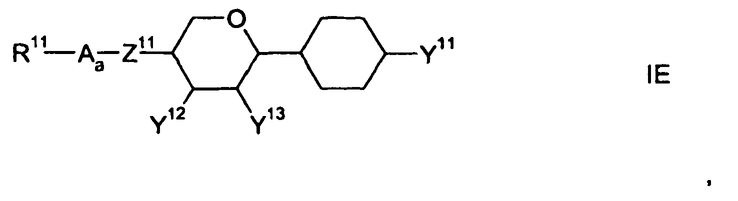 worin
R¹¹ H, F, Cl, Br, I, CN,
aromatische Kohlenwasserstoffreste mit vorzugsweise 6 bis 18 Kohlenstoffatomen, die gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂ oder C₁₋₁₅-Alkyl einfach oder mehrfach, gleich oder verschieden, substituiert sind,
heterocyclische Reste, in denen wenigstens ein Ringatom ein Heteroatom aus der Gruppe O, S und N besteht, wobei der Rest gegebenenfalls mit F, Cl, Br, I, CN, OH, SH, O-C₁₋₁₅-Alkyl, S-C₁₋₁₅-Alkyl, COOH, COO-C₁₋₁₅-Alkyl, Amino, NO₂, Aryl oder C₁₋₁₅-Alkyl einfach oder mehrfach, gleich oder verschieden, substituiert sein kann, oder
C₁₋₁₅-Alkyl bedeutet;
A für 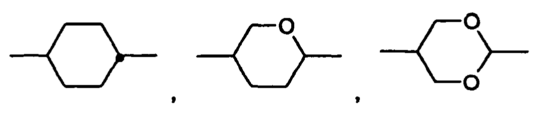 oder 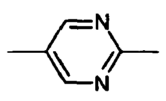 steht;
a 0, 1 oder 2 ist, wobei A gleiche oder verschiedene Bedeutungen annehmen kann, wenn a 2 ist;
Z¹¹ eine Einfachbindung, -CH₂-CH₂-, -CF₂-CF₂-, -CF₂-CH₂-, -CH₂-CF₂-, -CH₂-O-, -O-CH₂-, -CF₂-O- oder -O-CF₂- darstellt;
Y¹¹ -CO₂H oder -C(=S⁺R¹²)(-SR¹³)X⁻ bedeutet;
Y¹² und Y¹³ unabhängig voneinander H oder C₁₋₁₅-Alkyl bedeuten;
R¹² und R¹³ unabhängig voneinander einen unverzweigten oder verzweigten Alkylrest mit 1 bis 15 Kohlenstoffatomen bedeuten oder zusammen eine Einheit -(CH₂)ₚ- mit p = 2, 3, 4, 5 oder 6 bilden, wobei eine, zwei oder drei dieser CH₂-Gruppen mit wenigstens einem unverzweigten oder verzweigten Alkylrest mit 1 bis 8 Kohlenstoffatomen substituiert sein können; und
X⁻ ein schwach koordinierendes Anion ist;
**dadurch gekennzeichnet, daß** in einem Reaktionsschritt (E1)
(E1) die Verbindung der Formel ID1 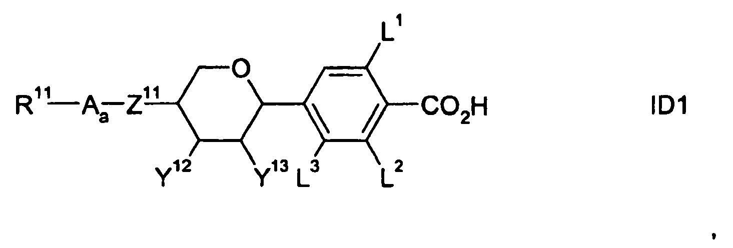 worin R¹¹, A, a, Z¹¹, Y¹² und Y¹³ wie oben für Formel IE definiert sind und L¹, L² und L³ H bedeuten,
mit Wasserstoff in Gegenwart eines Übergangsmetall-Katalysators in die Verbindung IE1 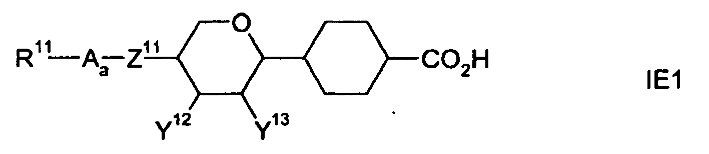 überführt wird;
und gegebenenfalls in einem Reaktionsschritt (E2)
(E2) die Verbindung der Formel IE1 in Gegenwart einer Säure HX mit HSR¹² und HSR¹³ oder mit HSR¹²R¹³SH in die Verbindung IE2 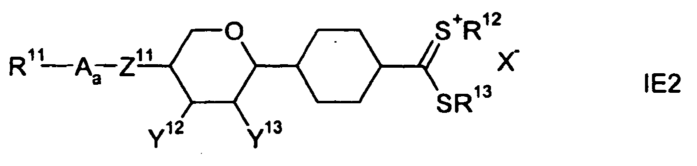
überführt wird.

## Claims

1. Compound of the general formula I 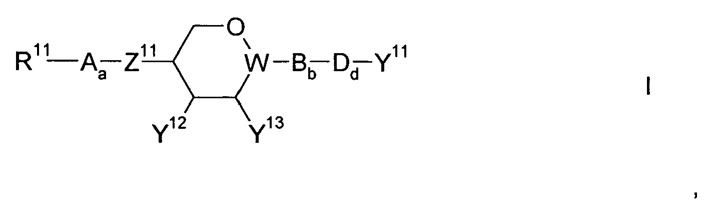 in which
R¹¹ denotes H, F, Cl, Br, I, CN,
aromatic hydrocarbon radicals, preferably having 6 to 18 carbon atoms, which are optionally mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂ or C₁₋₁₅-alkyl,
heterocyclic radicals in which at least one ring atom consists of a heteroatom from the group O, S and N, where the radical may optionally be mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂, aryl or C₁₋₁₅-alkyl, or
a halogenated or unsubstituted alkyl radical having 1 to 15 carbon atoms, where, in addition, one or more CH₂ groups in this radical may each be replaced, independently of one another, by -C≡C-, -CH=CH-, -O-, -CO-, -CO-O- or -O-CO- in such a way that O atoms are not linked directly to one another;
A stands for 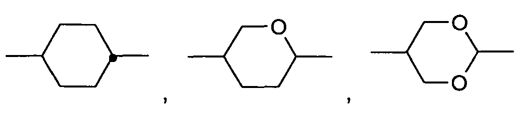 or 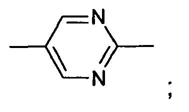
a is 0, 1 or 2;
Z¹¹ represents a single bond, -CH₂-CH₂-, -CF₂-CF₂-, -CF₂-CH₂-, -CH₂-CF₂-, -CH₂-O-, -O-CH₂-, -CF₂-O- or -O-CF₂-;
W denotes >CH- or >C=;
B and D, independently of one another, stand for 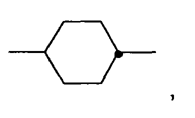 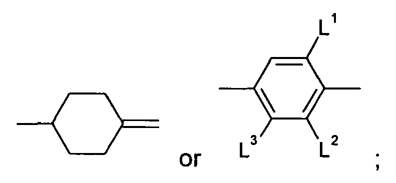
b and d, independently of one another, are 0 or 1;
Y¹¹ denotes =O, =C(SR¹²)(SR¹³), =CF₂, -H, -F, -Cl, -Br, -I, -CN, -OH, -SH, -CO-R¹⁴, -OSO₂R¹⁵, -C(=S⁺R¹²)(-SR¹³)X⁻, -B(OR¹⁶)(OR¹⁷), -BF₃⁻Cat⁺, -Si(OR¹⁸)(OR¹⁹)(OR²⁰) or alkyl, where alkyl denotes a halogenated or unsubstituted alkyl radical having 1 to 15 C atoms, in which, in addition, one or more CH₂ groups may each be replaced, independently of one another, by -C≡C-, -CH=CH-, -O-, -CO-, -CO-O- or -O-CO- in such a way that O atoms are not linked directly to one another;
Y¹² and Y¹³, independently of one another, denote H or alkyl, where alkyl denotes a halogenated or unsubstituted alkyl radical having 1 to 15 C atoms, in which, in addition, one or more CH₂ groups may each be replaced, independently of one another, by -C≡C-, -CH=CH-, -O-, -CO-, -CO-O- or -O-CO- in such a way that O atoms are not linked directly to one another;
L¹, L² and L³, independently of one another, denote H or F;
R¹² and R¹³, independently of one another, denote an unbranched or branched alkyl radical having 1 to 15 carbon atoms or together form a -(CH₂)ₚ- unit, where p = 2, 3, 4, 5 or 6, where one, two or three of these CH₂ groups may be substituted by at least one unbranched or branched alkyl radical having 1 to 8 carbon atoms;
R¹⁴ denotes OH, O-aryl, O-alkylaryl, O-C₁₋₁₅-alkyl, Cl, Br, aryl, aryl-C₁₋₁₅-alkyl or C₁₋₁₅-alkyl,
in which aryl denotes aromatic hydrocarbon radicals, preferably having 6 to 18 carbon atoms, which are optionally mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂ or C₁₋₁₅-alkyl;
R¹⁵ denotes aryl, aryl-C₁₋₁₅-alkyl or a halogenated or unsubstituted alkyl radical having 1 to 15 carbon atoms, where, in addition, one or more CH₂ groups in this alkyl radical may each be replaced, independently of one another, by -C≡C-, -CH=CH-, -O-, -CO-, -CO-O- or -O-CO- in such a way that O atoms are not linked directly to one another,
in which aryl denotes aromatic hydrocarbon radicals, preferably having 6 to 18 carbon atoms, which are optionally mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂ or C₁₋₁₅-alkyl;
R¹⁶ and R¹⁷ denote H or an unbranched or branched alkyl radical having 1 to 15 carbon atoms or together form a -(CH₂)ₚ- unit, where p = 2, 3, 4, 5 or 6, where one, two or three of these CH₂ groups may be substituted by at least one unbranched or branched alkyl radical having 1 to 8 carbon atoms;
R¹⁸, R¹⁹ and R²⁰, independently of one another, denote an unbranched or branched alkyl radical having 1 to 15 carbon atoms;
Cat⁺ is an alkali metal cation or a quaternary ammonium cation;
and
X is a weakly coordinating anion;
with the proviso
that W denotes >CH- if b+d ≠ 0;
that Y¹¹ does not denote =O, =C(SR¹²)(SR¹³) or =CF₂ if Y¹¹ is connected to B or 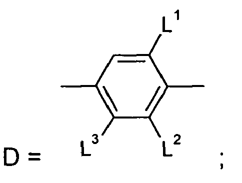 that Y¹¹ denotes -H, -I, -SH, -CO₂R¹⁴, -OSO₂R¹⁵, -C(=S⁺R¹²)(SR¹³)X⁻, -B(OR¹⁶)(OR¹⁷), -BF₃⁻Cat⁺, -Si(OR¹⁸)(OR¹⁹)(OR²⁰) or alkyl, where alkyl denotes a halogenated or unsubstituted alkyl radical having 1 to 15 C atoms, in which one or more CH₂ groups have each been replaced, independently of one another, by -C≡C-, -CH=CH-, -O-, -CO-, -CO-O- or -O-CO- in such a way that O atoms are not linked directly to one another and alkyl does not stand for alkoxy if W is connected directly to 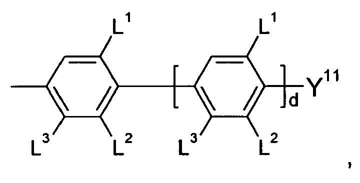 where d is 0 or 1; that B does not stand for 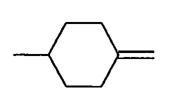 if d = 1; and that A can adopt identical or different meanings if a is 2.

2. Compound according to Claim 1, **characterised in that**
A stands for

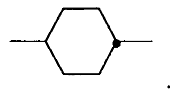

3. Compound according to Claim 1, **characterised in that**
a is 0.

4. Compound according to any one of Claims 1 to 3, **characterised in that**
Y¹² and Y¹³ denote H.

5. Compound according to any one of Claims 1 to 4, **characterised in that**
Z¹¹ represents a single bond, -CF₂O- or -OCF₂-.

6. Compound according to any one of Claims 1 to 5, **characterised in that**
R¹¹ denotes an unbranched halogenated or unsubstituted alkyl radical having 1 to 7 carbon atoms.

7. Compound according to any one of Claims 1 to 6, **characterised in that**
Y¹¹ denotes =O, =C(SR¹²)(SR¹³) or =CF₂.

8. Compound according to any one of Claims 1 to 6, **characterised in that**
Y¹¹ denotes -H, -F, -Cl, -Br, -I, -OH, -CO₂H, -C(=S⁺R¹²)(-SR¹³-)X⁻, -B(OR¹⁶)(OR¹⁷), -BF₃⁻Cat⁺ or -Si(OR¹⁸)(OR¹⁹)(OR²⁰).

9. Compound according to any one of Claims 1 to 6 and 8, **characterised in that**
X denotes BF₄⁻, CF₃SO₃⁻, C₄F₉SO₃⁻, PF₆⁻, SbF₆⁻ or AsF₆⁻.

10. Compound according to any one of Claims 1 to 9, **characterised in that**
b is 0 and d is 0.

11. Compound according to any one of Claims 1 to 9, **characterised in that**
b is 1 and d is 0.

12. Compound according to any one of Claims 1 to 9, **characterised in that**
b is 1 and d is 1.

13. Process for the preparation of a compound of the formula IA 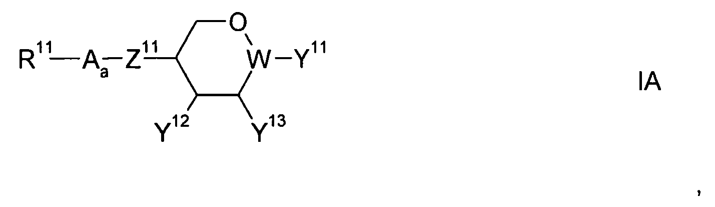 in which
R¹¹ denotes H, F, Cl, Br, I, CN,
aromatic hydrocarbon radicals, preferably having 6 to 18 carbon atoms, which are optionally mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂ or C₁₋₁₅-alkyl,
heterocyclic radicals in which at least one ring atom consists of a heteroatom from the group O, S and N, where the radical may optionally be mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂, aryl or C₁₋₁₅-alkyl, or
C₁₋₁₅-alkyl;
A stands for 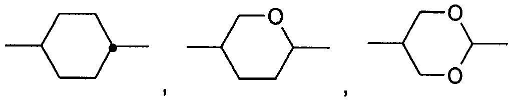 or 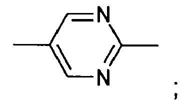
a is 0, 1 or 2, where A can adopt identical or different meanings if a is 2;
Z¹¹ represents a single bond, -CH₂-CH₂-, -CF₂-CF₂-, -CF₂-CH₂-, -CH₂-CF₂-, -CH₂-O-, -O-CH₂-, -CF₂-O- or -O-CF₂-;
W denotes >C=;
Y¹¹ denotes =O, =C(SR¹²)(SR¹³) or =CF₂;
Y¹² and Y¹³, independently of one another, denote H or C₁₋₁₅-alkyl;
and
R¹² and R¹³, independently of one another, denote an unbranched or branched alkyl radical having 1 to 15 carbon atoms or together form a -(CH₂)ₚ- unit, where p = 2, 3, 4, 5 or 6, where one, two or three of these CH₂ groups may be substituted by at least one unbranched or branched alkyl radical having 1 to 8 carbon atoms;
**characterised in that**
a compound of the formula II 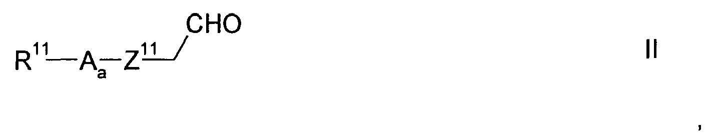 in which R¹¹, A, a and Z¹¹ are as defined above for the formula IA, is reacted in a reaction step (A1)
(A1) in the presence of a base with a compound of the formula III 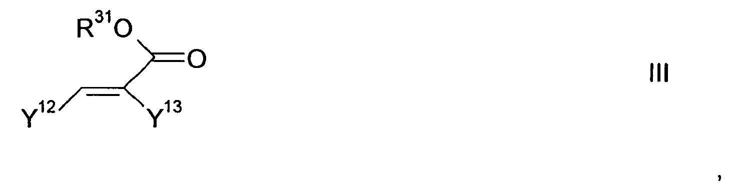 in which Y¹² and Y¹³ are as defined above for the formula IA, and R³¹ denotes an alkyl radical having 1 to 15 carbon atoms, to give a compound of the formula IV 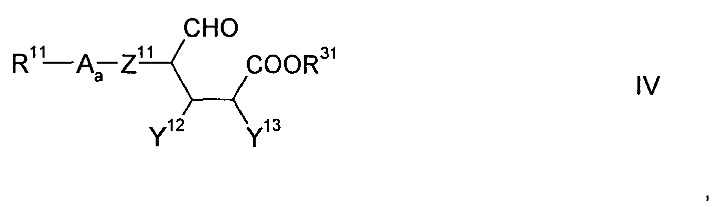 in which R¹¹, A, a, Z¹¹, Y¹² and Y¹³ are as defined above for the formula IA, and R³¹ is as defined above for the formula III;
and subsequently, in a reaction step (A2),
(A2) the compound of the formula IV is converted into the compound IA1 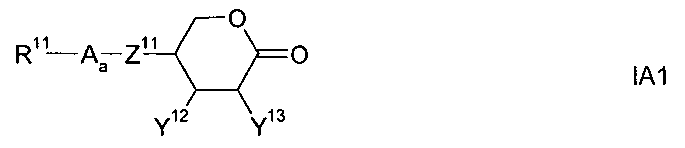 and optionally, in a reaction step (A3),
(A3) the compound of the formula IA1 is converted into the compound IA2 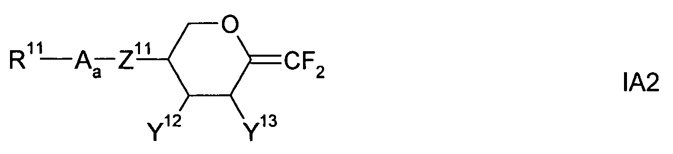 by reaction with CF₂Br₂ in the presence of P(N(R²¹)₂)₃, P(N(R²¹)₂)₂(OR²²) or P(N(R²¹)₂)(OR²²)₂, where R²¹ and R²², independently of one another, are an alkyl radical having 1 to 15 carbon atoms; or optionally, in a reaction step (A3'),
(A3') the compound of the formula IA1 is converted into the compound IA3 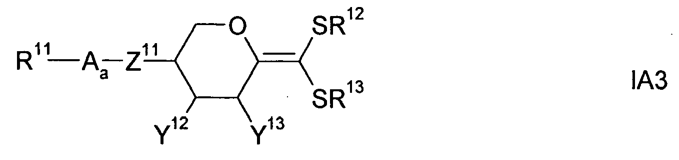 by reaction with CHG(SR¹²)(SR¹³), in which G denotes P(OCH₂R²³)₃, where R²³ is a perfluorinated alkyl radical having 1 to 5 carbon atoms, or Si(CH₃)₃ or Si(CH₂CH₃)₃, and R¹² and R¹³ are as defined above for the formula IA, in the presence of a strong base.

14. Process for the preparation of a compound of the formula IB 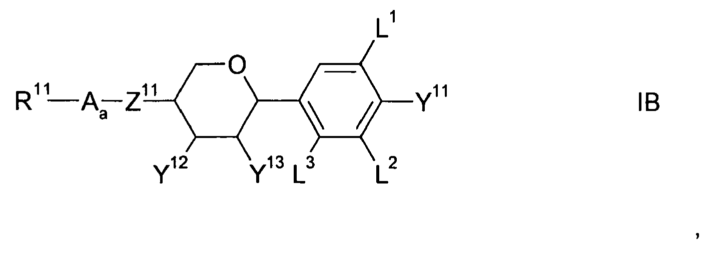 in which
R¹¹ denotes H, F, Cl, Br, I, CN,
aromatic hydrocarbon radicals, preferably having 6 to 18 carbon atoms, which are optionally mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂ or C₁₋₁₅-alkyl,
heterocyclic radicals in which at least one ring atom consists of a heteroatom from the group O, S and N, where the radical may optionally be mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂, aryl or C₁₋₁₅-alkyl, or
C₁₋₁₅-alkyl;
A stands for 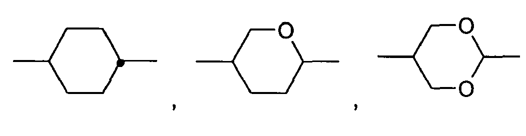 or 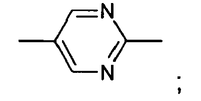
a is 0, 1 or 2, where A can adopt identical or different meanings if a is 2;
Z¹¹ represents a single bond, -CH₂-CH₂-, -CF₂-CF₂-, -CF₂-CH₂-, -CH₂-CF₂-, -CH₂-O-, -O-CH₂-, -CF₂-O- or -O-CF₂-;
Y¹¹ denotes -H, -F, -Cl, -Br, -I, -CN, -OH or -B(OR¹⁶)(OR¹⁷);
Y¹² and Y¹³, independently of one another, denote H or C₁₋₁₅-alkyl;
L¹, L² and L³, independently of one another, denote H or F; and
R¹⁶ and R¹⁷, independently of one another, denote H or an unbranched or branched alkyl radical having 1 to 15 carbon atoms or together form a -(CH₂)ₚ- unit, where p = 2, 3, 4, 5 or 6, where one, two or three of these CH₂ groups may be substituted by at least one unbranched or branched alkyl radical having 1 to 8 carbon atoms;
**characterised in that**,
in a reaction step (B1),
(B1) a compound of the formula IA1 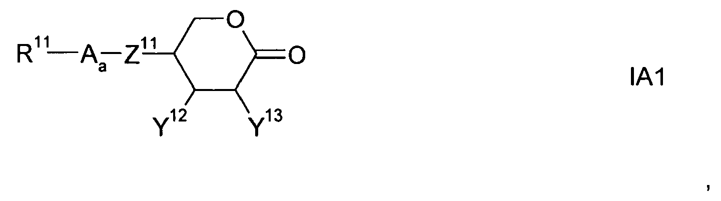 in which R¹¹, A, a, Z¹¹, Y¹² and Y¹³ are as defined above for the formula IB,
is reacted with a compound of the formula V 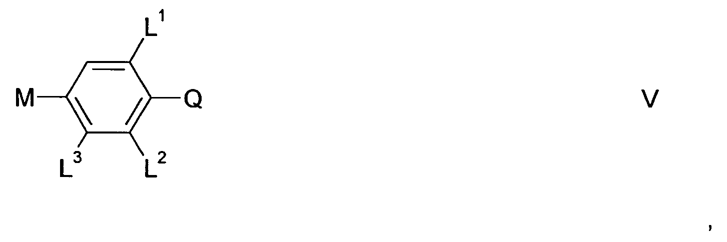 in which L¹, L² and L³ are as defined above for the formula IB, M denotes Li, Cl-Mg, Br-Mg or I-Mg, and Q denotes H, F, Cl, Br, I or CN, with formation of the compound of the formula IB1 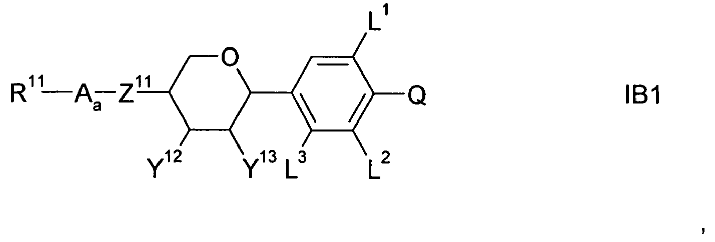 in which R¹¹, A, a, Z¹¹, Y¹², Y¹³, L¹, L² and L³ are as defined for the formula IB, and Q is as defined for the formula V;
and optionally, in a reaction step (B2),
(B2) the compound of the formula IB1 in which Q denotes Br is reacted with B(OR¹⁶)(OR¹⁷)(OR²⁴), where R¹⁶, R¹⁷ and R²⁴ denote an unbranched or branched alkyl radical having 1 to 15 carbon atoms, or with HB(OR¹⁶)(OR¹⁷), where R¹⁶ and R¹⁷ denote an unbranched or branched alkyl radical having 1 to 15 carbon atoms or together form a -(CH₂)ₚ- unit, where p = 2, 3, 4, 5 or 6, where one, two or three of these CH₂ groups may be substituted by at least one unbranched or branched alkyl radical having 1 to 8 carbon atoms, in the presence of an alkyllithium base,
to give the compound of the formula IB2 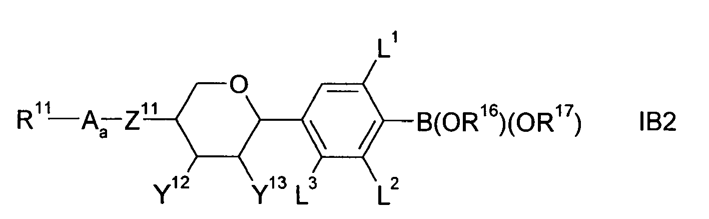 and optionally, in a reaction step (B3),
(B3) the compound IB2 is converted into the compound IB3 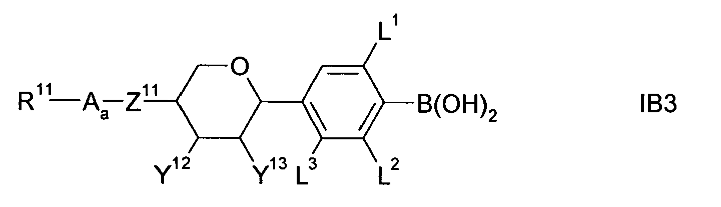 by reaction with an aqueous acid;
and/or optionally, in a reaction step (B4),
(B4) the compound IB2 or the compound IB3 is converted into the compound IB4 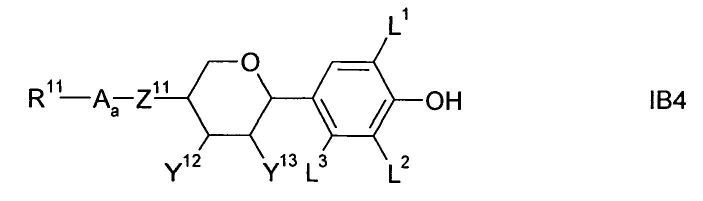 by reaction with hydrogen peroxide in alkaline or acidic solution.

15. Process for the preparation of a compound of the general formula IC 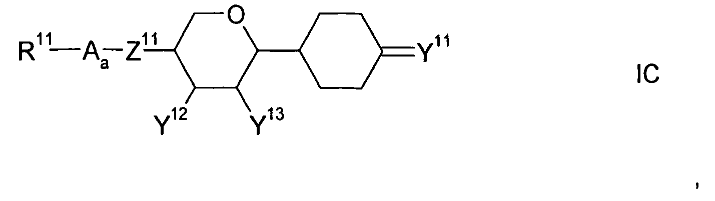 in which
R¹¹ denotes H, F, Cl, Br, I, CN,
aromatic hydrocarbon radicals, preferably having 6 to 18 carbon atoms, which are optionally mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂ or C₁₋₁₅-alkyl,
heterocyclic radicals in which at least one ring atom consists of a heteroatom from the group O, S and N, where the radical may optionally be mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂, aryl or C₁₋₁₅-alkyl, or
C₁₋₁₅-alkyl;
A stands for 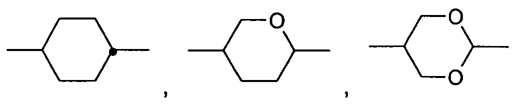 or 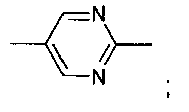
a is 0, 1 or 2, where A can adopt identical or different meanings if a is 2;
Z¹¹ represents a single bond, -CH₂-CH₂-, -CF₂-CF₂-, -CF₂-CH₂-, -CH₂-CF₂-, -CH₂-O-, -O-CH₂-, -CF₂-O- or -O-CF₂-;
Y¹¹ denotes =O, =C(SR¹²)(SR¹³) or =CF₂;
Y¹² and Y¹³, independently of one another, denote H or C₁₋₁₅-alkyl; and
R¹² and R¹³, independently of one another, denote an unbranched or branched alkyl radical having 1 to 15 carbon atoms or together form a -(CH₂)ₚ- unit, where p = 2, 3, 4, 5 or 6, where one, two or three of these CH₂ groups may be substituted by at least one unbranched or branched alkyl radical having 1 to 8 carbon atoms;
**characterised in that**, in a reaction step (C1),
(C1) the compound of the formula IB4 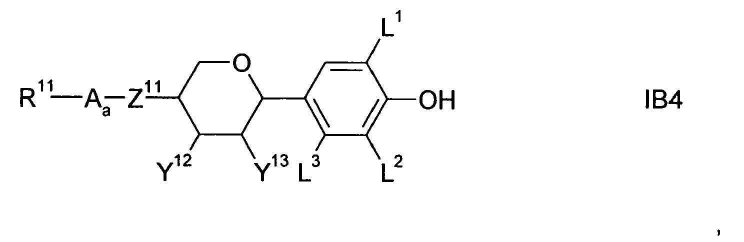 in which R¹¹, A, a, Z¹¹, Y¹² and Y¹³ are as defined above for the formula IC, and L¹, L² and L³ denote H,
is converted into the compound IC1 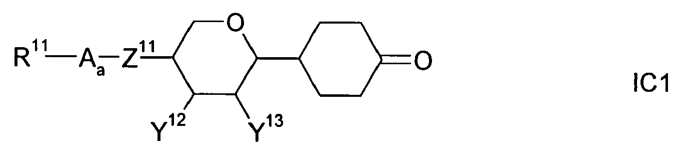 using hydrogen in the presence of a transition-metal catalyst;
and optionally, in a reaction step (C2),
(C2) the compound IC1 is converted into the compound IC2 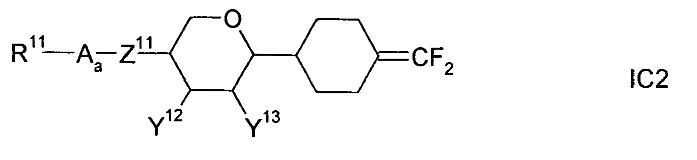 by reaction with CF₂Br₂ in the presence of P(N(R²¹)₂)₃, P(N(R²¹)₂)₂(OR²²) or P(N(R²¹)₂)(OR²²)₂, where R²¹ and R²², independently of one another, are an alkyl radical having 1 to 15 carbon atoms; or optionally, in a reaction step (C2'),
(C2') the compound of the formula IC1 is converted into the compound IC3 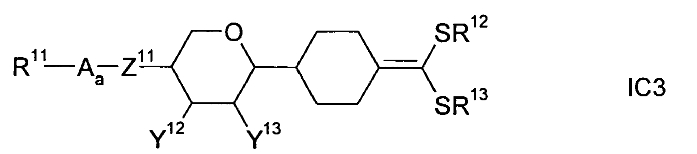 by reaction with CHG(SR¹²)(SR¹³), in which G denotes P(OCH₂R²³)₃, where R²³ is a perfluorinated alkyl radical having 1 to 5 carbon atoms, or Si(CH₃)₃ or Si(CH₂CH₃)₃, and R¹² and R¹³ are as defined above for the formula IC, in the presence of a strong base.

16. Process for the preparation of a compound of the formula ID 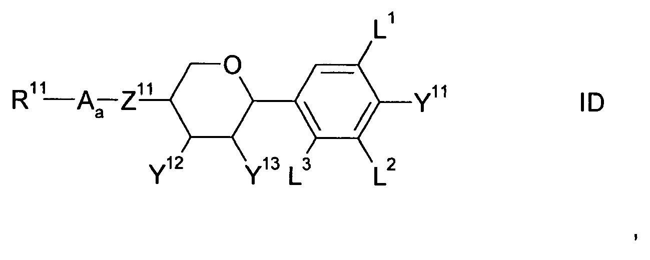 in which
R¹¹ denotes H, F, Cl, Br, I, CN,
aromatic hydrocarbon radicals, preferably having 6 to 18 carbon atoms, which are optionally mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂ or C₁₋₁₅-alkyl,
heterocyclic radicals in which at least one ring atom consists of a heteroatom from the group O, S and N, where the radical may optionally be mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂, aryl or C₁₋₁₅-alkyl, or
C₁₋₁₅-alkyl;
A stands for 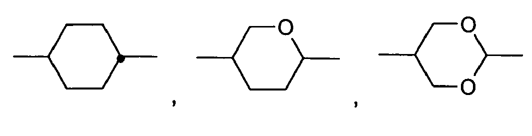 or 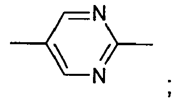
a is 0, 1 or 2, where A can adopt identical or different meanings if a is 2;
Z¹¹ represents a single bond, -CH₂-CH₂-, -CF₂-CF₂-, -CF₂-CH₂-, -CH₂-CF₂-, -CH₂-O-, -O-CH₂-, -CF₂-O- or -O-CF₂-;
Y¹¹ denotes -CO₂H or -C(=S⁺R¹²)(-SR¹³)X⁻;
Y¹² and Y¹³, independently of one another, denote H or C₁₋₁₅-alkyl;
L¹, L² and L³, independently of one another, denote H or F;
R¹² and R¹³, independently of one another, denote an unbranched or branched alkyl radical having 1 to 15 carbon atoms or together form a -(CH₂)ₚ- unit, where p = 2, 3, 4, 5 or 6, where one, two or three of these CH₂ groups may be substituted by at least one unbranched or branched alkyl radical having 1 to 8 carbon atoms; and
X is a weakly coordinating anion;
**characterised in that**, in a reaction step (D1),
(D1) a compound of the formula IB1 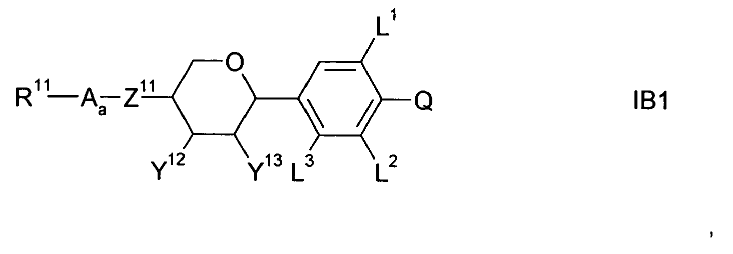 in which R¹¹, A, a, Z¹, Y¹², Y¹³, L¹, L² and L³ are as defined for the formula ID, and Q denotes H or Br,
is reacted with an organometallic base and CO₂ to give the compound ID1 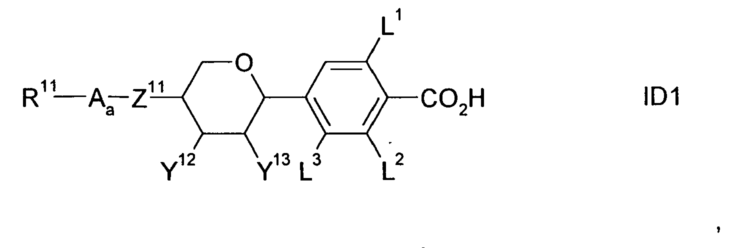 in which R¹¹, A, a, Z¹¹, Y¹², Y¹³, L¹, L² and L³ are as defined for the formula ID;
and optionally, in a reaction step (D2),
(D2) the compound ID1 is converted into the compound ID2 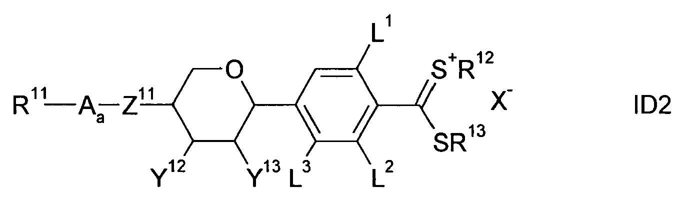 in the presence of an acid HX using HSR¹² and HSR¹³ or using HSR¹²R¹³SH.

17. Process for the preparation of a compound of the formula IE 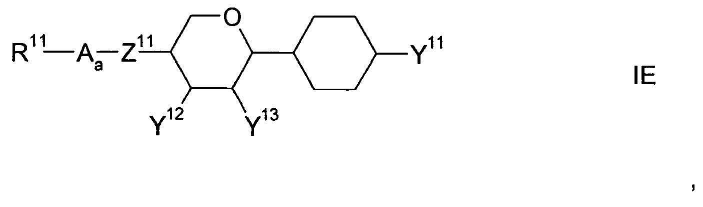 in which
R¹¹ denotes H, F, Cl, Br, I, CN,
aromatic hydrocarbon radicals, preferably having 6 to 18 carbon atoms, which are optionally mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂ or C₁₋₁₅-alkyl,
heterocyclic radicals in which at least one ring atom consists of a heteroatom from the group O, S and N, where the radical may optionally be mono- or polysubstituted, identically or differently, by F, Cl, Br, I, CN, OH, SH, O-C₁₋₁₅-alkyl, S-C₁₋₁₅-alkyl, COOH, COO-C₁₋₁₅-alkyl, amino, NO₂, aryl or C₁₋₁₅-alkyl, or
C₁₋₁₅-alkyl;
A stands for 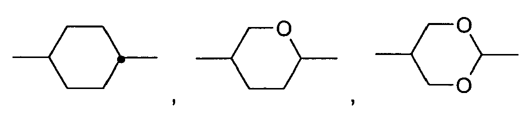 or 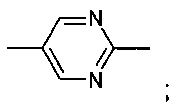
a is 0, 1 or 2, where A can adopt identical or different meanings if a is 2;
Z¹¹ represents a single bond, -CH₂-CH₂-, -CF₂-CF₂-, -CF₂-CH₂-, -CH₂-CF₂-, -CH₂-O-, -O-CH₂-, -CF₂-O-or-O-CF₂-;
Y¹¹ denotes -CO₂H or -C(=S⁺R¹²)(-SR¹³)X⁻;
Y¹² and Y¹³, independently of one another, denote H or C₁₋₁₅-alkyl;
R¹² and R¹³, independently of one another, denote an unbranched or branched alkyl radical having 1 to 15 carbon atoms or together form a -(CH₂)ₚ- unit, where p = 2, 3, 4, 5 or 6, where one, two or three of these CH₂ groups may be substituted by at least one unbranched or branched alkyl radical having 1 to 8 carbon atoms; and
X⁻ is a weakly coordinating anion;
**characterised in that**, in a reaction step (E1),
(E1) the compound of the formula ID1 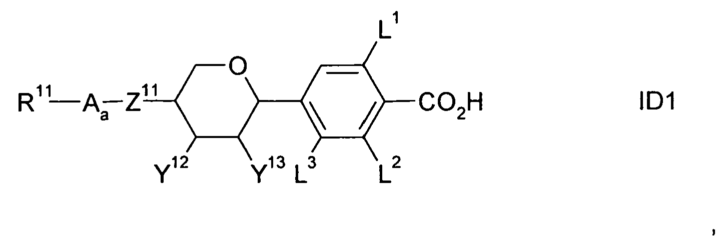 in which R¹¹, A, a, Z¹¹, Y¹² and Y¹³ are as defined above for the formula IE, and L¹, L² and L³ denote H,
is converted into the compound IE1 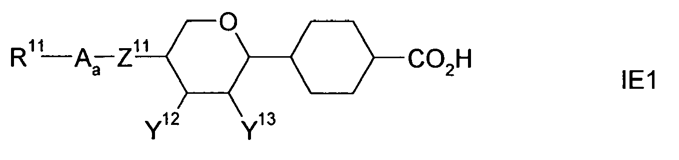 using hydrogen in the presence of a transition-metal catalyst;
and optionally, in a reaction step (E2),
(E2) the compound of the formula IE1 is converted into the compound IE2 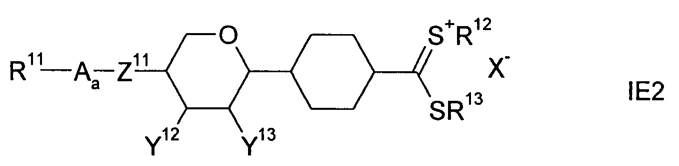 in the presence of an acid HX using HSR¹² and HSR¹³ or using HSR¹²R¹³SH.

## Revendications

1. Composé de formule générale I 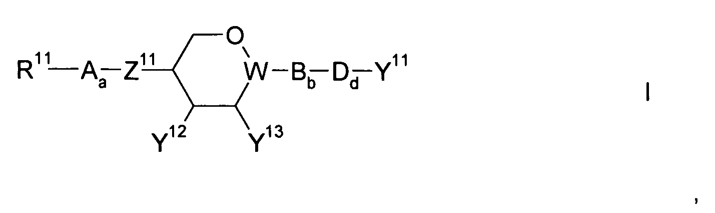 dans laquelle
R¹¹ désigne H, F, Cl, Br, I, CN,
des radicaux hydrocarbonés aromatiques, ayant de préférence de 6 à 18 atomes de carbone, qui sont éventuellement mono- ou polysubstitués, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂ ou alkyl(C₁₋₁₅), des radicaux hétérocycliques dans lesquels au moins un atome de cycle est constitué d'un hétéroatome issu du groupe O, S et N, où le radical peut éventuellement être mono- ou polysubstitué, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂, aryle ou alkyl(C₁₋₁₅), ou
un radical alkyle halogéné ou non substitué ayant de 1 à 15 atomes de carbone, où, en outre, un ou plusieurs groupements CH₂ dans ce radical peuvent chacun être remplacés, indépendamment les uns des autres, par -C≡C-, -CH=CH-, -O-, -CO-, -CO-O- ou -O-CO- de telle sorte que les atomes de O ne soient pas directement liés les uns aux autres ;
A représente 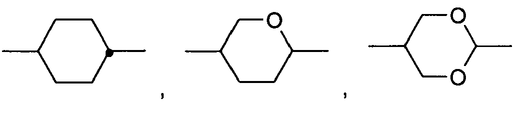 ou 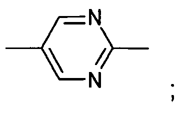
a vaut 0, 1 ou 2 ;
Z¹¹ représente une liaison simple, -CH₂-CH₂-, -CF₂-CF₂-, -CF₂-CH₂-, -CH₂-CF₂-, -CH₂-O-, -O-CH₂-, -CF₂-O- ou -O-CF₂- ;
W désigne >CH- ou >C= ;
B et D, indépendamment l'un de l'autre, représentent 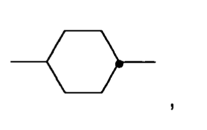 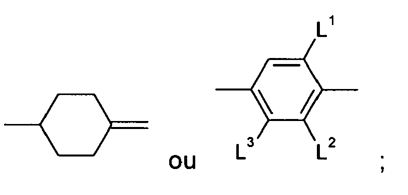
b et d, indépendamment l'un de l'autre, valent 0 ou 1 ;
Y¹¹ désigne =O, =C(SR¹²)(SR¹³), =CF₂, -H, -F, -Cl, -Br, -I, -CN, -OH, -SH, -CO-R¹⁴, -OSO₂R¹⁵, -C(=S⁺R¹²)(-SR¹³)X⁻, -B(OR¹⁶)(OR¹⁷), -BF₃⁻Cat⁺, -Si(OR¹⁸)(OR¹⁹)(OR²⁰) ou alkyle, où alkyle désigne un radical alkyle halogéné ou non substitué ayant de 1 à 15 atomes de C, dans lequel, en outre, un ou plusieurs groupements CH₂ peuvent chacun être remplacés, indépendamment les uns des autres, par -C≡C-, -CH=CH-, -O-, -CO-, -CO-O- ou -O-CO- de telle sorte que les atomes de O ne soient pas directement liés les uns aux autres ;
Y¹² et Y¹³, indépendamment l'un de l'autre, désignent H ou alkyle, où alkyle désigne un radical alkyle halogéné ou non substitué ayant de 1 à 15 atomes de C, dans lequel, en outre, un ou plusieurs groupements CH₂ peuvent chacun être remplacés, indépendamment les uns des autres, par -C≡C-, -CH=CH-, -O-, -CO-, -CO-O- ou -O-CO- de telle sorte que les atomes de O ne soient pas directement liés les uns aux autres ;
L¹, L² et L³, indépendamment l'un de l'autre, désignent H ou F ;
R¹² et R¹³, indépendamment l'un de l'autre, désignent un radical alkyle non ramifié ou ramifié ayant de 1 à 15 atomes de carbone ou forment ensemble un motif -(CH₂)ₚ-, où p = 2, 3, 4, 5 ou 6, où un, deux ou trois parmi ces groupements CH₂ peuvent être substitués par au moins un radical alkyle non ramifié ou ramifié ayant de 1 à 8 atomes de carbone ;
R¹⁴ désigne OH, O-aryle, O-alkylaryle, O-alkyl(C₁₋₁₅), Cl, Br, aryle, aryl-alkyl(C₁₋₁₅) ou alkyl(C₁₋₁₅),
dans lequel aryle désigne des radicaux hydrocarbonés aromatiques, ayant de préférence de 6 à 18 atomes de carbone, qui sont éventuellement mono- ou polysubstitués, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂ ou alkyl(C₁₋₁₅) ;
R¹⁵ désigne aryle, aryl-alkyl(C₁₋₁₅) ou un radical alkyle halogéné ou non substitué ayant de 1 à 15 atomes de carbone, où, en outre, un ou plusieurs groupements CH₂ dans ce radical alkyle peuvent chacun être remplacés, indépendamment les uns des autres, par -C≡C-, -CH=CH-, -O-, -CO-, -CO-O- ou -O-CO- de telle sorte que les atomes de O ne soient pas directement liés les uns aux autres,
dans lequel aryle désigne des radicaux hydrocarbonés aromatiques, ayant de préférence de 6 à 18 atomes de carbone, qui sont éventuellement mono- ou polysubstitués, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂ ou alkyl(C₁₋₁₅) ;
R¹⁶ et R¹⁷ désignent H ou un radical alkyle non ramifié ou ramifié ayant de 1 à 15 atomes de carbone ou forment ensemble un motif -(CH₂)ₚ-, où p = 2, 3, 4, 5 ou 6, où un, deux ou trois parmi ces groupements CH₂ peuvent être substitués par au moins un radical alkyle non ramifié ou ramifié ayant de 1 à 8 atomes de carbone ;
R¹⁸, R¹⁹ et R²⁰, indépendamment l'un de l'autre, désignent un radical alkyle non ramifié ou ramifié ayant de 1 à 15 atomes de carbone ;
Cat⁺ est un cation de métal alcalin ou un cation d'ammonium quaternaire ;
et
X⁻ est un anion à coordination faible ;
à condition
que W désigne >CH- si b+d ≠ 0 ;
que Y¹¹ ne désigne pas =O, =C(SR¹²)(SR¹³) ou =CF₂ si Y¹¹ est lié à B ou 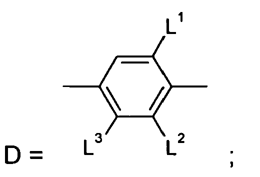 que Y¹¹ désigne -H, -I, -SH, -CO₂R¹⁴, -OSO₂R¹⁵, -C(=S⁺R¹²)(SR¹³)X⁻, -B(OR¹⁶)(OR¹⁷), -BF₃⁻Cat⁺, -Si(OR¹⁸)(OR¹⁹)(OR²⁰) ou alkyle, où alkyle désigne un radical alkyle halogéné ou non substitué ayant de 1 à 15 atomes de C, dans lequel un ou plusieurs groupements CH₂ ont chacun été remplacés, indépendamment les uns des autres, par -C≡C-, -CH=CH-, -O-, -CO-, -CO-O- ou -O-CO- de telle sorte que les atomes de O ne soient pas directement liés les uns aux autres et alkyle ne représente pas alcoxy si W est relié directement à 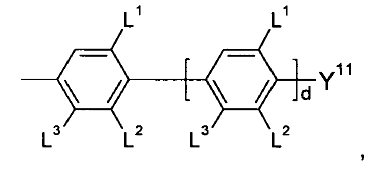 où d vaut 0 ou 1 ;
que B ne représente pas 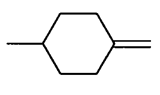 sid=1;et
que A puisse revêtir des significations identiques ou différentes si a vaut 2.

2. Composé selon la revendication 1, **caractérisé en ce que**
A représente

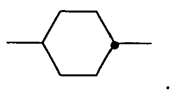

3. Composé selon la revendication 1, **caractérisé en ce que**
a vaut 0.

4. Composé selon l'une quelconque des revendications 1 à 3, **caractérisé en ce que**
Y¹² et Y¹³ désignent H.

5. Composé selon l'une quelconque des revendications 1 à 4, **caractérisé en ce que**
Z¹¹ représente une liaison simple, -CF₂O- ou -OCF₂-.

6. Composé selon l'une quelconque des revendications 1 à 5, **caractérisé en ce que**
R¹¹ désigne un radical alkyle non substitué ou halogéné non ramifié ayant de 1 à 7 atomes de carbone.

7. Composé selon l'une quelconque des revendications 1 à 6, **caractérisé en ce que**
Y¹¹ désigne =O, =C(SR¹²)(SR¹³) ou =CF₂.

8. Composé selon l'une quelconque des revendications 1 à 6, **caractérisé en ce que**
Y¹¹ désigne -H, -F, -Cl, -Br, -I, -OH, -CO₂H, -C(=S⁺R¹²)(-SR¹³)X⁻, -B(OR¹⁶)(OR¹⁷), -BF₃⁻Cat⁺ ou -Si(OR¹⁸)(OR¹⁹)(OR²⁰).

9. Composé selon l'une quelconque des revendications 1 à 6 et 8,
**caractérisé en ce que**
X⁻ désigne BF₄⁻, CF₃SO₃⁻, C₄F₉SO₃⁻, PF₆⁻, SbF₆⁻ ou AsF₆⁻.

10. Composé selon l'une quelconque des revendications 1 à 9, **caractérisé en ce que**
b vaut 0 et d vaut 0.

11. Composé selon l'une quelconque des revendications 1 à 9, **caractérisé en ce que**
b vaut 1 et d vaut 0.

12. Composé selon l'une quelconque des revendications 1 à 9, **caractérisé en ce que**
b vaut 1 et d vaut 1.

13. Procédé de préparation d'un composé de formule IA 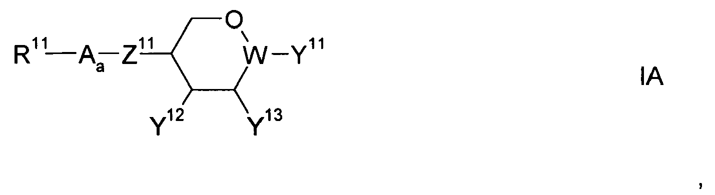 dans laquelle
R¹¹ désigne H, F, Cl, Br, I, CN,
des radicaux hydrocarbonés aromatiques, ayant de préférence de 6 à 18 atomes de carbone, qui sont éventuellement mono- ou polysubstitués, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂ ou alkyl(C₁₋₁₅),
des radicaux hétérocycliques dans lesquels au moins un atome de cycle est constitué d'un hétéroatome issu du groupe O, S et N, où le radical peut éventuellement être mono- ou polysubstitué, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂, aryle ou alkyl(C₁₋₁₅), ou
alkyl(C₁₋₁₅) ;
A représente 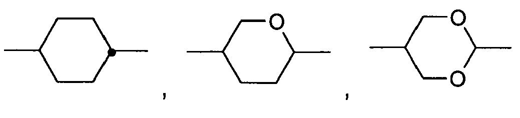 ou 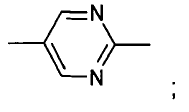
a vaut 0, 1 ou 2, où A peut revêtir des significations identiques ou différentes si a vaut 2 ;
Z¹¹ représente une liaison simple, -CH₂-CH₂-, -CF₂-CF₂-, -CF₂-CH₂-, -CH₂-CF₂-, -CH₂-O-, -O-CH₂-, -CF₂-O- ou -O-CF₂- ;
W désigne >C= ;
Y¹¹ désigne =O, =C(SR¹²)(SR¹³) ou =CF₂;
Y¹² et Y¹³, indépendamment l'un de l'autre, désignent H ou alkyl(C₁₋₁₅) ; et
R¹² et R¹³, indépendamment l'un de l'autre, désignent un radical alkyle non ramifié ou ramifié ayant de 1 à 15 atomes de carbone ou forment ensemble un motif -(CH₂)ₚ-, où p = 2, 3, 4, 5 ou 6, où un, deux ou trois parmi ces groupements CH₂ peuvent être substitués par au moins un radical alkyle non ramifié ou ramifié ayant de 1 à 8 atomes de carbone ;
**caractérisé en ce que**
un composé de formule II 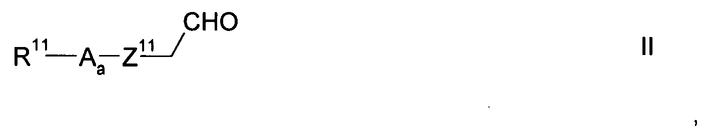 dans laquelle R¹¹, A, a et Z¹¹ sont tels que définis ci-dessus pour la formule IA,
est réagi dans une étape de réaction (A1)
(A1) en présence d'une base avec un composé de formule III 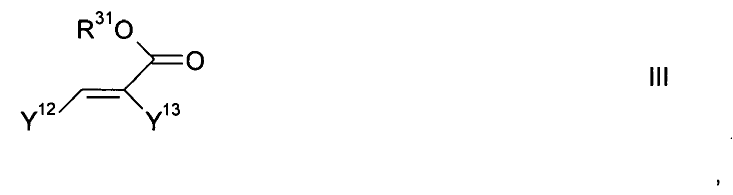 dans laquelle Y¹² et Y¹³ sont tels que définis ci-dessus pour la formule IA, et R³¹ désigne un radical alkyle ayant de 1 à 15 atomes de carbone, pour donner un composé de formule IV 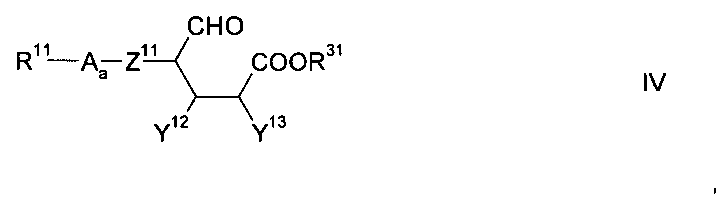 dans laquelle R¹¹, A, a, Z¹¹, Y¹² et Y¹³ sont tels que définis ci-dessus pour la formule IA, et R³¹ est tel que défini ci-dessus pour la formule III ;
et ensuite, dans une étape de réaction (A2),
(A2) le composé de formule IV est converti en composé IA1 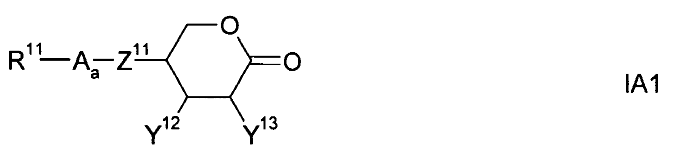 et éventuellement, dans une étape de réaction (A3),
(A3) le composé de formule IA1 est converti en composé IA2 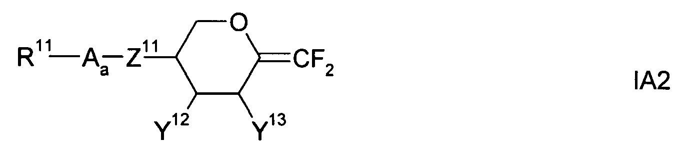 par une réaction avec CF₂Br₂ en présence de P(N(R²¹)₂)₃, P(N(R²¹)₂)₂(OR²²) ou P(N(R²¹)₂)(OR²²)₂, où R²¹ et R²², indépendamment l'un de l'autre, sont un radical alkyle ayant de 1 à 15 atomes de carbone ;
ou éventuellement, dans une étape de réaction (A3'),
(A3') le composé de formule IA1 est converti en composé IA3 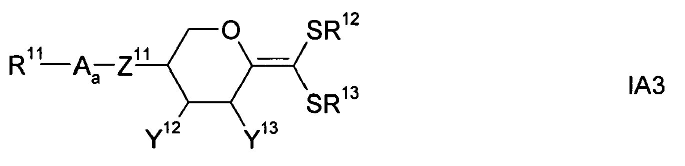 par une réaction avec CHG(SR¹²)(SR¹³), dans lequel G désigne P(OCH₂R²³)₃, où R²³ est un radical alkyle perfluoré ayant de 1 à 5 atomes de carbone, ou Si(CH₃)₃ ou Si(CH₂CH₃)₃, et R¹² et R¹³ sont tels que définis ci-dessus pour la formule IA, en présence d'une base forte.

14. Procédé de préparation d'un composé de formule IB 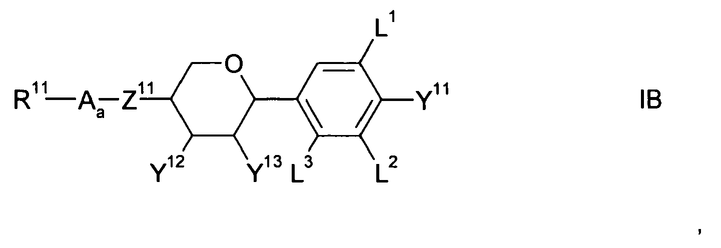 dans laquelle
R¹¹ désigne H, F, Cl, Br, I, CN,
des radicaux hydrocarbonés aromatiques, ayant de préférence de 6 à 18 atomes de carbone, qui sont éventuellement mono- ou polysubstitués, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂ ou alkyl(C₁₋₁₅), des radicaux hétérocycliques dans lesquels au moins un atome de cycle est constitué d'un hétéroatome issu du groupe O, S et N, où le radical peut éventuellement être mono- ou polysubstitué, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂, aryle ou alkyl(C₁₋₁₅), ou
alkyl(C₁₋₁₅) ;
A représente 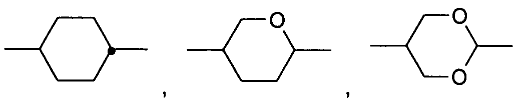 ou 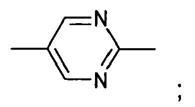
a vaut 0, 1 ou 2, où A peut revêtir des significations identiques ou différentes si a vaut 2 ;
Z¹¹ représente une liaison simple, -CH₂-CH₂-, -CF₂-CF₂-, -CF₂-CH₂-, -CH₂-CF₂-, -CH₂-O-, -O-CH₂-, -CF₂-O- ou -O-CF₂- ;
Y¹¹ désigne -H, -F, -Cl, -Br, -I, -CN, -OH ou -B(OR¹⁶)(OR¹⁷) ;
Y¹² et Y¹³, indépendamment l'un de l'autre, désignent H ou alkyl(C₁₋₁₅) ;
L¹, L² et L³, indépendamment l'un de l'autre, désignent H ou F ; et
R¹⁶ et R¹⁷, indépendamment l'un de l'autre, désignent H ou un radical alkyle non ramifié ou ramifié ayant de 1 à 15 atomes de carbone ou forment ensemble un motif -(CH₂)ₚ-, où p = 2, 3, 4, 5 ou 6, où un, deux ou trois parmi ces groupements CH₂ peuvent être substitués par au moins un radical alkyle non ramifié ou ramifié ayant de 1 à 8 atomes de carbone ;
**caractérisé en ce que**,
dans une étape de réaction (B1),
(B1) un composé de formule IA1 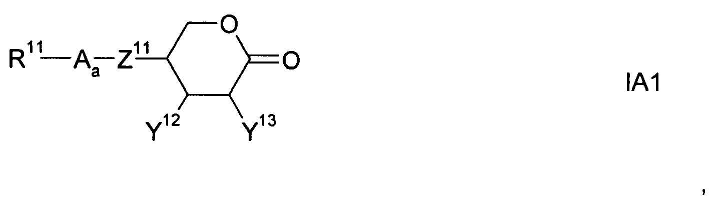 dans laquelle R¹¹, A, a, Z¹¹, Y¹² et Y¹³ sont tels que définis ci-dessus pour la formule IB,
est réagi avec un composé de formule V 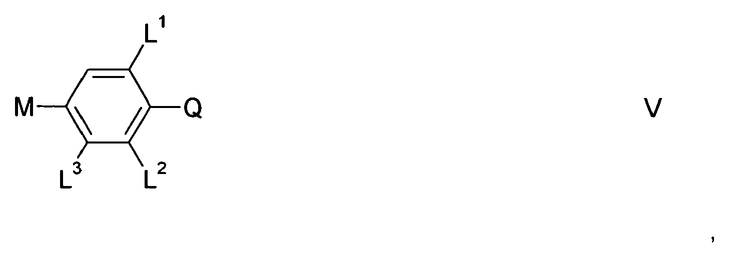 dans laquelle L¹, L² et L³ sont tels que définis ci-dessus pour la formule IB, M désigne Li, Cl-Mg, Br-Mg ou I-Mg, et Q désigne H, F, Cl,
Br, I ou CN, avec la formation du composé de formule IB1 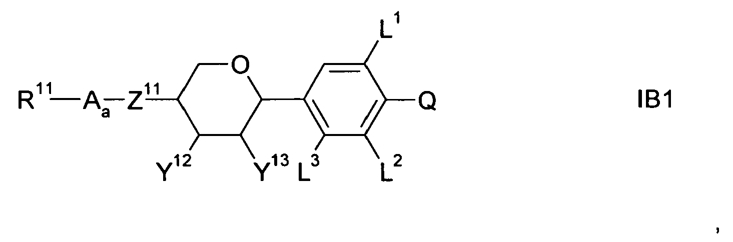 dans laquelle R¹¹, A, a, Z¹¹, Y¹², Y¹³, L¹, L² et L³ sont tels que définis pour la formule IB, et Q est tel que défini pour la formule V ;
et éventuellement, dans une étape de réaction (B2),
(B2) le composé de formule IB1 dans laquelle Q désigne Br est réagi avec B(OR¹⁶)(OR¹⁷)(OR²⁴), où R¹⁶, R¹⁷ et R²⁴ désignent un radical alkyle non ramifié ou ramifié ayant de 1 à 15 atomes de carbone, ou avec HB(OR¹⁶)(OR¹⁷), où R¹⁶ et R¹⁷ désignent un radical alkyle non ramifié ou ramifié ayant de 1 à 15 atomes de carbone ou forment ensemble un motif -(CH₂)ₚ-, où p = 2, 3, 4, 5 ou 6, où un, deux ou trois parmi ces groupements CH₂ peuvent être substitués par au moins un radical alkyle non ramifié ou ramifié ayant de 1 à 8 atomes de carbone, en présence d'une base d'alkyllithium,
pour donner le composé de formule IB2 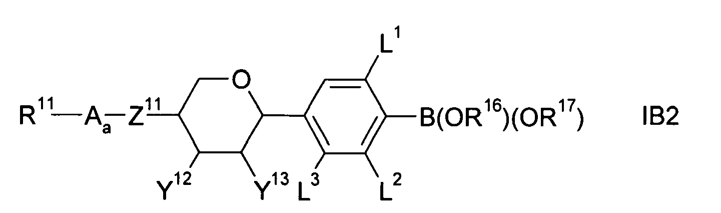 et éventuellement, dans une étape de réaction (B3),
(B3) le composé IB2 est converti en composé IB3 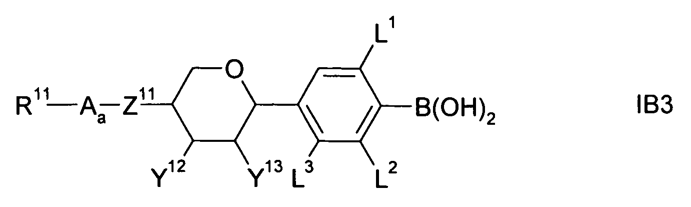 par une réaction avec un acide aqueux ;
et/ou éventuellement, dans une étape de réaction (B4),
(B4) le composé IB2 ou le composé IB3 est converti en composé IB4 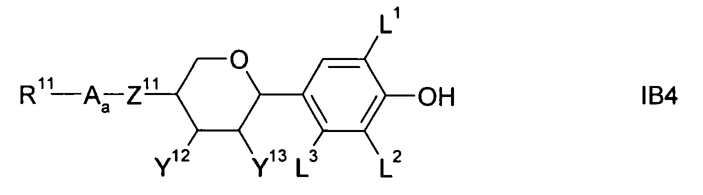 par une réaction avec du peroxyde d'hydrogène en solution alcaline ou acide.

15. Procédé de préparation d'un composé de formule générale IC 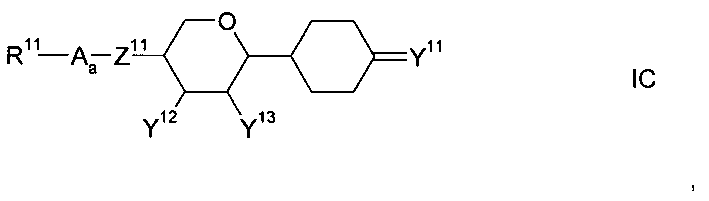 dans laquelle
R¹¹ désigne H, F, Cl, Br, I, CN,
des radicaux hydrocarbonés aromatiques, ayant de préférence de 6 à 18 atomes de carbone, qui sont éventuellement mono- ou polysubstitués, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂ ou alkyl(C₁₋₁₅), des radicaux hétérocycliques dans lesquels au moins un atome de cycle est constitué d'un hétéroatome issu du groupe O, S et N, où le radical peut éventuellement être mono- ou polysubstitué, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂, aryle ou alkyl(C₁₋₁₅), ou
alkyl(C₁₋₁₅) ;
A représente 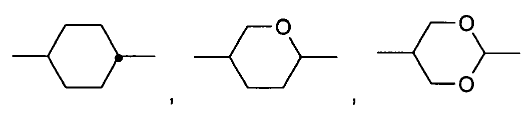 ou 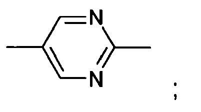
a vaut 0, 1 ou 2, où A peut revêtir des significations identiques ou différentes si a vaut 2 ;
Z¹¹ représente une liaison simple, -CH₂-CH₂-, -CF₂-CF₂-, -CF₂-CH₂-, -CH₂-CF₂-, -CH₂-O-, -O-CH₂-, -CF₂-O- ou -O-CF₂- ;
Y¹¹ désigne =O, =C(SR¹²)(SR¹³) ou =CF₂ ;
Y¹² et Y¹³, indépendamment l'un de l'autre, désignent H ou alkyl(C₁₋₁₅) ; et
R¹² et R¹³, indépendamment l'un de l'autre, désignent un radical alkyle non ramifié ou ramifié ayant de 1 à 15 atomes de carbone ou forment ensemble un motif -(CH₂)ₚ-, où p = 2, 3, 4, 5 ou 6, où un, deux ou trois parmi ces groupements CH₂ peuvent être substitués par au moins un radical alkyle non ramifié ou ramifié ayant de 1 à 8 atomes de carbone ;
**caractérisé en ce que**, dans une étape de réaction (C1),
(C1) le composé de formule IB4 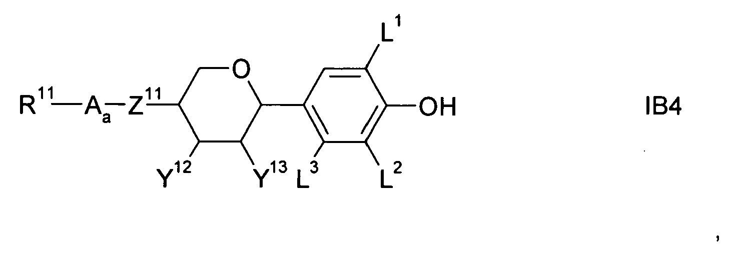 dans laquelle R¹¹, A, a, Z¹¹, Y¹² et Y¹³ sont tels que définis ci-dessus pour la formule IC, et L¹, L² et L³ désignent H,
est converti en composé IC1 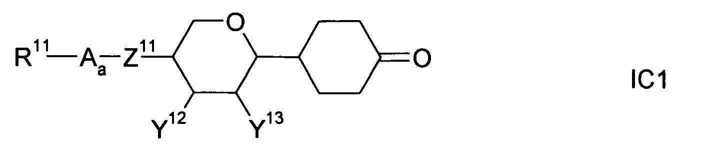 en utilisant de l'hydrogène en présence d'un catalyseur à base d'un métal de transition ;
et éventuellement, dans une étape de réaction (C2),
(C2) le composé IC1 est converti en composé IC2 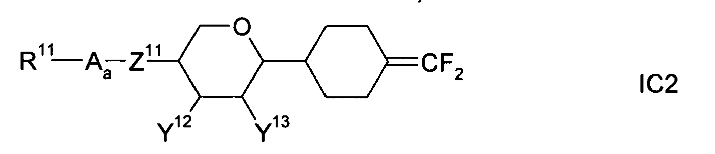 par une réaction avec CF₂Br₂ en présence de P(N(R²¹)₂)₃, P(N(R²¹)₂)₂(OR²²) ou P(N(R²¹)₂)(OR²²)₂, où R²¹ et R²², indépendamment l'un de l'autre, sont un radical alkyle ayant de 1 à 15 atomes de carbone ;
ou éventuellement, dans une étape de réaction (C2'),
(C2') le composé de formule IC1 est converti en composé IC3 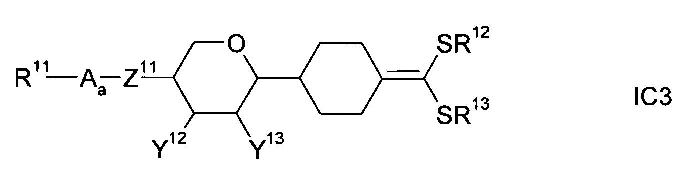 par une réaction avec CHG(SR¹²)(SR¹³), dans lequel G désigne P(OCH₂R²³)₃, où R²³ est un radical alkyle perfluoré ayant de 1 à 5 atomes de carbone, ou Si(CH₃)₃ ou Si(CH₂CH₃)₃, et R¹² et R¹³ sont tels que définis ci-dessus pour la formule IC, en présence d'une base forte.

16. Procédé de préparation d'un composé de formule ID 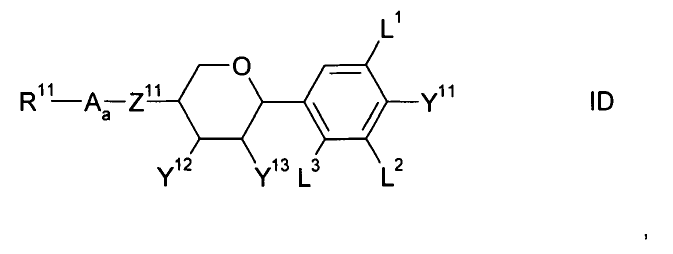 dans laquelle
R¹¹ désigne H, F, Cl, Br, I, CN,
des radicaux hydrocarbonés aromatiques, ayant de préférence de 6 à 18 atomes de carbone, qui sont éventuellement mono- ou polysubstitués, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂ ou alkyl(C₁₋₁₅), des radicaux hétérocycliques dans lesquels au moins un atome de cycle est constitué d'un hétéroatome issu du groupe O, S et N, où le radical peut éventuellement être mono- ou polysubstitué, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂, aryle ou alkyl(C₁₋₁₅), ou
alkyl(C₁₋₁₅) ;
A représente 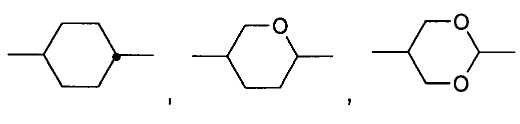 ou 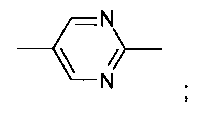
a vaut 0, 1 ou 2, où A peut revêtir des significations identiques ou différentes si a vaut 2 ;
Z¹¹ représente une liaison simple, -CH₂-CH₂-, -CF₂-CF₂-, -CF₂-CH₂-, -CH₂-CF₂-, -CH₂-O-, -O-CH₂-, -CF₂-O- ou -O-CF₂- ;
Y¹¹ désigne -CO₂H ou -C(=S⁺R¹²)(-SR¹³)X⁻ ;
Y¹² et Y¹³, indépendamment l'un de l'autre, désignent H ou alkyl(C₁₋₁₅) ;
L¹, L² et L³, indépendamment l'un de l'autre, désignent H ou F ;
R¹² et R¹³, indépendamment l'un de l'autre, désignent un radical alkyle non ramifié ou ramifié ayant de 1 à 15 atomes de carbone ou forment ensemble un motif -(CH₂)ₚ-, où p = 2, 3, 4, 5 ou 6, où un, deux ou trois parmi ces groupements CH₂ peuvent être substitués par au moins un radical alkyle non ramifié ou ramifié ayant de 1 à 8 atomes de carbone ; et
X⁻ est un anion à coordination faible ;
**caractérisé en ce que**, dans une étape de réaction (D1),
(D1) un composé de formule IB1 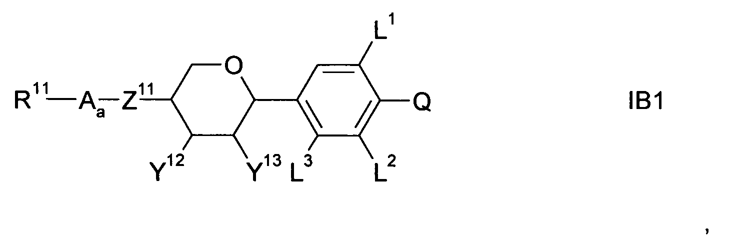 dans laquelle R¹¹, A, a, Z¹¹, Y¹², Y¹³, L¹, L² et L³ sont tels que définis pour la formule ID, et Q désigne H ou Br,
est réagi avec une base organométallique et CO₂ pour donner le composé ID1 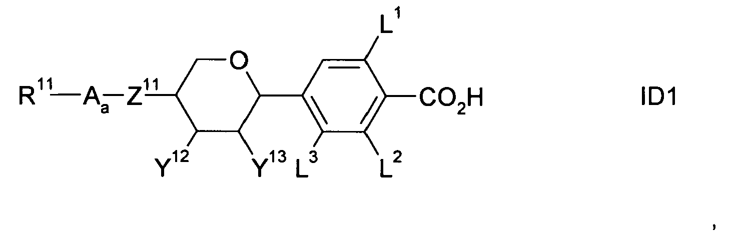 dans lequel R¹¹, A, a, Z¹¹, Y¹², Y¹³, L¹, L² et L³ sont tels que définis pour la formule ID ;
et éventuellement, dans une étape de réaction (D2),
(D2) le composé ID1 est converti en composé ID2 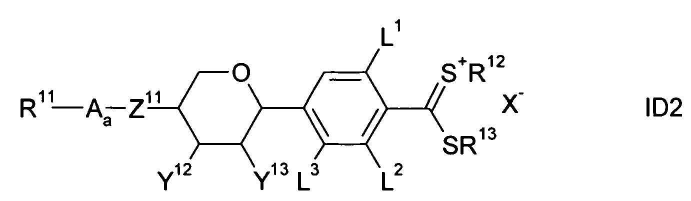 en présence d'un acide HX en utilisant HSR¹² et HSR¹³ ou en utilisant HSR¹²R¹³SH.

17. Procédé de préparation d'un composé de formule IE 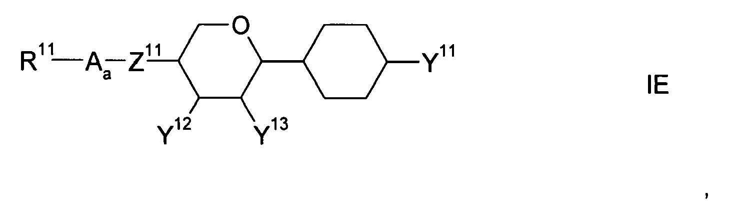 dans laquelle
R¹¹ désigne H, F, Cl, Br, I, CN,
des radicaux hydrocarbonés aromatiques, ayant de préférence de 6 à 18 atomes de carbone, qui sont éventuellement mono- ou polysubstitués, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂ ou alkyl(C₁₋₁₅), des radicaux hétérocycliques dans lesquels au moins un atome de cycle est constitué d'un hétéroatome issu du groupe O, S et N, où le radical peut éventuellement être mono- ou polysubstitué, de manière identique ou différente, par F, Cl, Br, I, CN, OH, SH, O-alkyl(C₁₋₁₅), S-alkyl(C₁₋₁₅), COOH, COO-alkyl(C₁₋₁₅), amino, NO₂, aryle ou alkyl(C₁₋₁₅), ou
alkyl(C₁₋₁₅) ;
A représente 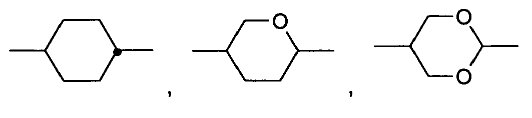 ou 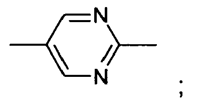
a vaut 0, 1 ou 2, où A peut revêtir des significations identiques ou différentes si a vaut 2 ;
Z¹¹ représente une liaison simple, -CH₂-CH₂-, -CF₂-CF₂-, -CF₂-CH₂-, -CH₂-CF₂-, -CH₂-O-, -O-CH₂-, -CF₂-O- ou -O-CF₂- ;
Y¹¹ désigne -CO₂H ou -C(=S⁺R¹²)(-SR¹³)X⁻;
Y¹² et Y¹³, indépendamment l'un de l'autre, désignent H ou alkyl(C₁₋₁₅) ;
R¹² et R¹³, indépendamment l'un de l'autre, désignent un radical alkyle non ramifié ou ramifié ayant de 1 à 15 atomes de carbone ou forment ensemble un motif -(CH₂)p-, où p = 2, 3, 4, 5 ou 6, où un, deux ou trois parmi ces groupements CH₂ peuvent être substitués par au moins un radical alkyle non ramifié ou ramifié ayant de 1 à 8 atomes de carbone ; et
X est un anion à coordination faible ;
**caractérisé en ce que**, dans une étape de réaction (E1),
(E1) le composé de formule ID1 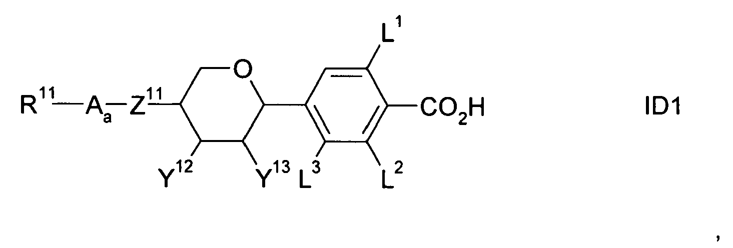 dans laquelle R¹¹, A, a, Z¹¹, Y¹² et Y¹³ sont tels que définis ci-dessus pour la formule IE, et L¹, L² et L³ désignent H,
est converti en composé IE1 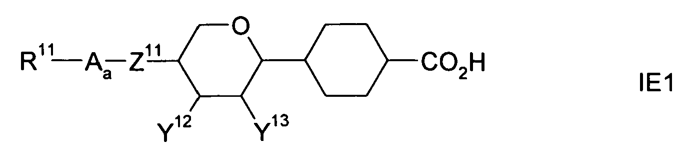 en utilisant de l'hydrogène en présence d'un catalyseur à base d'un métal de transition ;
et éventuellement, dans une étape de réaction (E2),
(E2) le composé de formule IE1 est converti en composé IE2 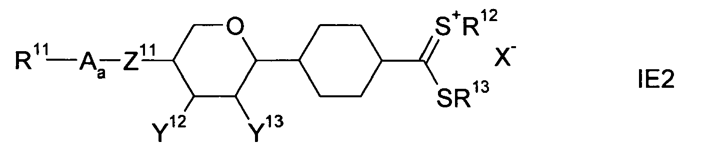 en présence d'un acide HX en utilisant HSR¹² et HSR¹³ ou en utilisant HSR¹²R¹³SH.
